(19) Europäisches Patentamt / European Patent Office / Office européen des brevets

(11) **EP 4 256 310 B1**

(12) **EUROPEAN PATENT SPECIFICATION**

(45) Date of publication and mention of the grant of the patent:
**17.06.2026 Bulletin 2026/25**

(21) Application number: **21840736.9**

(22) Date of filing: **03.12.2021**

(51) International Patent Classification (IPC):
$G01N\ 21/65^{(2006.01)}$  $A61B\ 5/00^{(2006.01)}$

(52) Cooperative Patent Classification (CPC):
**G01N 21/65; A61B 5/0075; A61B 5/0088;
A61B 5/168; A61B 5/201; A61B 5/4082;
A61B 5/4088; A61B 5/448; A61B 5/449;
A61B 5/7267; A61B 5/7275; G16H 10/40;
G16H 40/63; G16H 40/67; G16H 50/20;**  (Cont.)

(86) International application number:
**PCT/US2021/061885**

(87) International publication number:
**WO 2022/120225 (09.06.2022 Gazette 2022/23)**

(54) **SYSTEMS AND METHODS FOR DYNAMIC RAMAN PROFILING OF BIOLOGICAL DISEASES AND DISORDERS**

SYSTEME UND VERFAHREN ZUR DYNAMISCHEN RAMAN-PROFILIERUNG BIOLOGISCHER ERKRANKUNGEN UND STÖRUNGEN

SYSTÈMES ET PROCÉDÉS POUR PROFILAGE RAMAN DYNAMIQUE DE MALADIES ET DE TROUBLES BIOLOGIQUES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **04.12.2020 US 202063121800 P**

(43) Date of publication of application:
**11.10.2023 Bulletin 2023/41**

(73) Proprietor: **Icahn School of Medicine at Mount Sinai
New York, NY 10029 (US)**

(72) Inventors:
 • **ARORA, Manish
   New York, NY 10029 (US)**
 • **CURTIN, Paul
   New York, NY 10029 (US)**
 • **AUSTIN, Christine
   New York, NY 10029 (US)**

(74) Representative: **Grund, Martin
   Grund Intellectual Property Group
   Patentanwälte und Solicitor PartG mbB
   Steinsdorfstraße 2
   80538 München (DE)**

(56) References cited:
 CN-A- 110 320 184    CN-A- 110 763 844
 US-A1- 2005 283 058

 • **PUDNEY PAUL D ET AL: "Confocal Raman Spectroscopy of Whole Hairs", APPLIED SPECTROSCOPY, 1 December 2013 (2013-12-01), XP55885959, Retrieved from the Internet <URL:https://www.researchgate.net/profile/ Paul-Pudney/publication/259446389_Confocal_ Raman_Spectroscopy_of_Whole_Hairs/links/ 0046352d5723b5c897000000/ Confocal-Raman-Spectroscopy-of-Whole-Hairs. pdf> [retrieved on 20220201], DOI: 10.1366/ 13-07086.Source:**

- ZHANG GUOJIN ET AL: "Measuring changes in chemistry, composition, and molecular structure within hair fibers by infrared and Raman spectroscopic imaging", JOURNAL OF BIOMEDICAL OPTICS, vol. 16, no. 5, 1 January 2011 (2011-01-01), 1000 20th St. Bellingham WA 98225-6705 USA, pages 056009, XP55886371, ISSN: 1083-3668, DOI: 10.1117/1.3580286
- AUSTIN CHRISTINE ET AL: "Uncovering system-specific stress signatures in primate teeth with multimodal imaging", SCIENTIFIC REPORTS, vol. 6, no. 1, 1 May 2016 (2016-05-01), XP055884415, Retrieved from the Internet <URL:https://www.nature.com/articles/srep18802.pdf> DOI: 10.1038/srep18802
- MORISHITA HIROFUMI ET AL: "Tooth-Matrix Biomarkers to Reconstruct Critical Periods of Brain Plasticity", TRENDS IN NEUROSCIENCES, vol. 40, no. 1, January 2017 (2017-01-01), pages 1 - 3, XP029873898, ISSN: 0166-2236, DOI: 10.1016/J.TINS.2016.11.003
- WEI XIAOLI ET AL: "Comparison of hair from rectum cancer patients and from healthy persons by Raman microspectroscopy and imaging", JOURNAL OF MOLECULAR STRUCTURE, vol. 1048, 2013, pages 83 - 87, XP028685273, ISSN: 0022-2860, DOI: 10.1016/J.MOLSTRUC.2013.05.005
- ZHANG GUOJIN ET AL: "Measuring changes in chemistry, composition, and molecular structure within hair fibers by infrared and Raman spectroscopic imaging", JOURNAL OF BIOMEDICAL OPTICS, vol. 16, no. 5, 1 January 2011 (2011-01-01), 1000 20th St. Bellingham WA 98225-6705 USA, pages 056009, XP055886371, ISSN: 1083-3668, DOI: 10.1117/1.3580286
- PUDNEY PAUL D ET AL: "Confocal Raman Spectroscopy of Whole Hairs", APPLIED SPECTROSCOPY, 1 December 2013 (2013-12-01), XP055885959, Retrieved from the Internet <URL:https://www.researchgate.net/profile/Paul-Pudney/publication/259446389_Confocal_Raman_Spectroscopy_of_Whole_Hairs/links/0046352d5723b5c897000000/Confocal-Raman-Spectroscopy-of-Whole-Hairs.pdf> [retrieved on 20220201], DOI: 10.1366/13-07086.Source:

(52) Cooperative Patent Classification (CPC): (Cont.)
**G16H 50/70;** A61B 5/0022; A61B 5/0071; A61B 2503/06

## Description

## BACKGROUND

Dynamic biological responses may be indicative of underlying biological processes having structural and functional significance for humans. For example, aberrant or abnormal dynamic biological response may be associated with many biological conditions, such as diseases and disorders. Examples of such biological conditions may include neurological conditions (e.g., autism spectrum disorder, schizophrenia, or attention-deficit/hyperactivity disorder (ADHD)), neurodegenerative conditions (e.g., amyotrophic lateral sclerosis (ALS), Alzheimer's disease, Parkinson's disease, and Huntington's disease), and cancers (e.g., pediatric cancer).

It is known that Raman spectral imaging of a tooth surface may be used to identify changes in enamel and dentin elemental concentrations, (Austin C. et al. (2016),"Uncovering system-specific stress signatures in primate teeth with multimodal imaging", Nature Scientific Reports, vol: 6(1):1-11. Further, monitoring tooth biomarkers with e.g. multiplexed mass-spectrometry in order to measure changes in brain plasticity and corresponding regulators of brain plasticity is described in Morishita H. et al. (2017), "Tooth-Matrix Biomarkers to Reconstruct Critical Periods of Brain Plasticity", TRENDS IN NEUROSCIENCE, vol:40(1):1-3.

## SUMMARY

The invention is defined by the appended claims.

Thus, in a first aspect, the invention relates to a device comprising one or more processors, and memory storing one or more programs for execution by the one or more processors, the one or more programs comprising instructions for: (a) sampling each respective position in a plurality of positions along a reference line on a tooth sample obtained from a subject associated with a Raman signature of the subject, thereby obtaining a plurality of Raman spectra, each Raman spectrum in the plurality of Raman spectra corresponding to a different position in the plurality of positions, and each position in the plurality of positions representing a different period of growth of the tooth sample associated with the Raman signature; (b) analyzing each of the plurality of Raman spectra across the reference line on the tooth sample thereby obtaining a first dataset; (c) deriving a respective second dataset of a set of features from the plurality of Raman spectra, each respective feature in the set of features being determined by a sequential variation in the Raman spectra of the plurality of Raman spectra measurements; and (d) processing the set of features using a trained model to predict a subject's diagnostic status with respect to disease or disorder associated with the Raman signature, wherein the disease or disorder comprises autism spectrum disorder (ASD) or amyotrophic lateral sclerosis (ALS).

In a second aspect, the invention relates to a non-transitory computer readable storage medium and one or more computer programs embedded therein for classification, the one or more computer programs comprising instructions which, when executed by a computer system, cause the computer system to perform a method comprising: (a) sampling each respective position in a plurality of positions along a reference line on a tooth sample obtained from a subject associated with a Raman signature of the subject, thereby obtaining a plurality of Raman spectra, each Raman spectra in the plurality of Raman spectra corresponding to a different position in the plurality of positions, and each position in the plurality of positions representing a different period of growth of the tooth sample associated with the Raman signature; (b) analyzing each of the plurality of Raman spectra across the reference line on the tooth sample thereby obtaining a first dataset; (c) deriving a respective second dataset of a set of features from the plurality of Raman spectra, each respective feature in the set of features being determined by a sequential variation in the Raman spectra of the plurality of Raman spectra measurements; and (d) processing the set of features using a trained model to predict a subject's diagnostic status with respect to disease or disorder associated with the Raman signature, wherein the disease or disorder comprises autism spectrum disorder (ASD) or amyotrophic lateral sclerosis (ALS).

## BRIEF DESCRIPTION OF THE DRAWINGS

The novel features of the invention are set forth in the appended claims. A better understanding of the features and advantages of the present invention will be obtained by reference to the following detailed description that sets forth illustrative embodiments, in which the principles of the invention are utilized, and the accompanying drawings (also "Figure" and "FIG." herein), of which:

FIG. 1 shows an example of a block diagram of a computing device 100 of the present disclosure.
FIGS. 2A-2C show illustrations of a hair sample (FIG. 2A), a tooth sample (FIG. 2B), and a nail sample (FIG. 2C) of a subject.
FIG. 3 shows a flow chart of a method 300 for evaluating a subject for a biological condition.
FIG. 4 shows a computer system that is programmed or otherwise configured to implement methods provided herein.

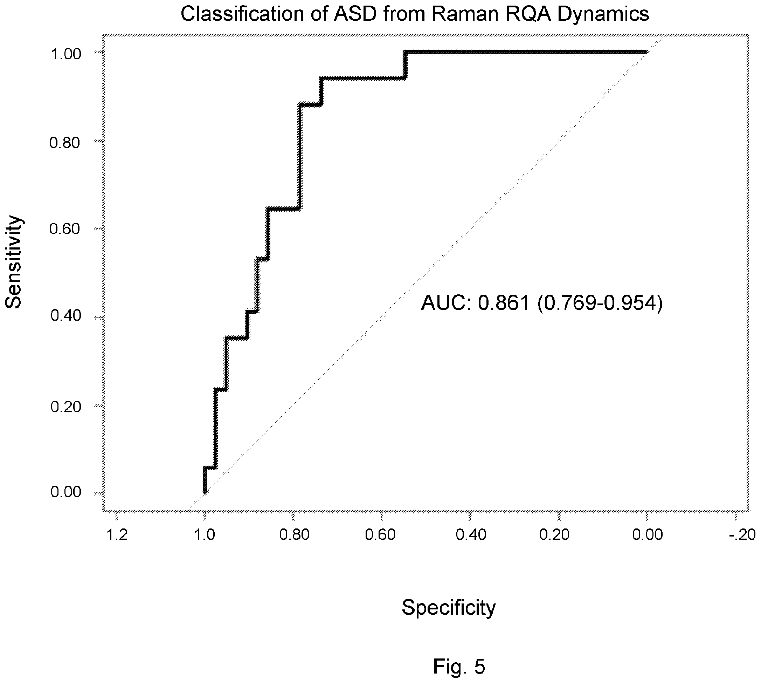

FIG. 5 shows an example of model accuracy for predicting diagnostic status for autism spectrum disorder (ASD) utilizing features derived from application of RQA to ICA-derived dimensions of the Raman waveform, as indicated by an experimental Receiver Operating Characteristics (ROC) curve for evaluating accuracy of the disclosed method of evaluating a subject for autism spectrum disorder. Device performance is measured by calculating the area-under-the-curve (AUC) of the ROC plot, which provides a measure of performance at varying classification thresholds; here, the AUC was 0.86, indicating robustly accurate predictive performance.

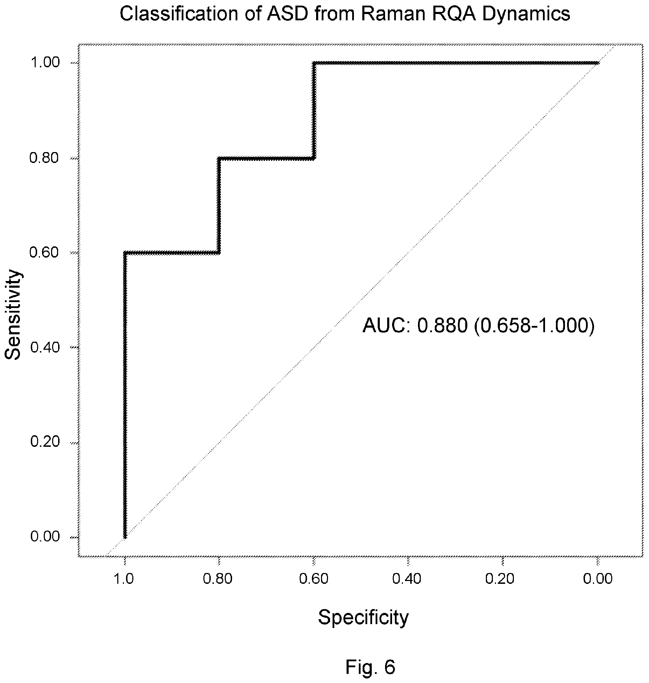

**FIG.** 6 shows an example of model accuracy for predicting diagnostic status for amyotrophic lateral sclerosis (ALS) utilizing features derived from application of RQA to ICA-derived dimensions of the Raman waveform, as indicated by an experimental Receiver Operating Characteristics (ROC) curve for evaluating accuracy of the disclosed method of evaluating a subject for autism spectrum disorder. Device performance is measured by calculating the area-under-the-curve (AUC) of the ROC plot, which provides a measure of performance at varying classification thresholds; here, the AUC was 0.88, indicating robustly accurate predictive performance.

## DETAILED DESCRIPTION

Dynamic biological responses may be indicative of underlying biological processes having structural and functional significance for humans. For example, aberrant or abnormal dynamic biological response may be associated with many biological conditions, such as diseases and disorders. Examples of such biological conditions may include neurological conditions (e.g., autism spectrum disorder, schizophrenia, or attention-deficit/hyperactivity disorder (ADHD)), neurodegenerative conditions (e.g., amyotrophic lateral sclerosis (ALS), Alzheimer's disease, Parkinson's disease, and Huntington's disease), and cancers (e.g., pediatric cancer).

Given the above background, there is a need for accurate methods and systems for the diagnosis of biological conditions, and especially for non-invasive diagnosis. Such diagnosis may be based on accurate profiling of biomarkers detectable with non-invasive methods for diagnosis of the biological conditions. The present disclosure provides improved systems and methods for accurate diagnosis of biological conditions based on analysis of dynamic biological response data from non-invasively obtained biological samples from subjects. Such improved systems and methods for accurate diagnosis of biological conditions may be based on a combination of Raman profiling of biological samples and artificial intelligence data analysis. The present disclosure addresses these needs, for example, by providing a biological sample biomarker for diagnosis of biological conditions. The biological sample includes a human biological specimen that is associated with incremental growth. Such a biological sample could be a hair shaft, a tooth, and a nail. The non-invasive biomarker of the present disclosure can be used for the diagnosis of young children, even infants younger than one year old. In some cases, the child may be between the ages of about 12 and about 5 years old. In some embodiments, the child may be less than about 12, 11, 10, 9, 8, 7, 5, 4, 3, 2, or 1 year(s) old. In some embodiments, the child may be at least about 1, 2, 3, 4, 5, 6, 7, 8, 9, 10, 11, or 12 year(s) old.

Not claimed but for illustrative purposes only, the present disclosure provides a method for predicting a subject's diagnostic status with respect to a disease or disorder, comprising: (a) exposing a biological sample of the subject to a light source, where the biological sample comprises a tooth sample, a hair sample, or a nail sample; (b) acquiring a plurality of Raman spectra from the exposed biological sample; (c) processing the plurality of Raman spectra to generate a spatial map of the plurality of Raman spectra; and (d) predicting a subject's diagnostic status with respect to a disease or disorder based at least in part on the spatial map of the plurality of Raman spectra. The light source may comprise a laser.

The analyzing may determine temporal dynamics of underlying biological processes. In some examples, the analyzing comprises reducing the dimensionality of the plurality of Raman spectra (e.g., by independent components analysis) prior to the processing. In some examples, the optical signal is generated by a light source (e.g., a laser). In some examples, the biological sample comprises the tooth sample. In some examples, the method further comprises detecting or monitoring changes in a temporal stress profile that are indicative of a temporal response of the subject. In some examples, the temporal response comprises a biochemical response. In some examples, the temporal response comprises a biological response, a physiological response, an anatomical response, a treatment response, a stress-related response, or a combination thereof. In some examples, the plurality of Raman spectra comprises from about 200 to about 3700 wave numbers. In some examples, the acquiring comprises using Raman spectroscopy microscope. In some examples, the Raman spectroscopy microscope comprises an 50X air coupled objective, 63X water immersion coupled objection, or any combination thereof. In some examples, the laser comprises a wavelength of about 785 nm, a wavelength of about 532 nm, or any combination thereof. In some examples, the acquiring is performed using an integration time of about 0.2 seconds to about 0.3 seconds. In some examples, the acquiring comprises moving the biological sample with a step size of about 2 microns to about 5 microns, subsequent to acquiring a Raman spectrum of the plurality of Raman spectra.

The systems and methods disclosed herein may use Raman Spectroscopy alone, or in combination with other techniques. Such techniques may include laser ablation-inductively coupled plasma-mass spectrometry (LA-ICP-MS), C-reactive immunohistochemistry fluorescence staining, and others. Combining techniques may improve diagnostic accuracy or precision of a given technique alone. The addition of LA-ICP-MS may provide a plurality of non-invasive metal metabolism biomarkers of a given biological sample that may complement the diagnostic power of Raman Spectroscopy.

The metal metabolism biomarkers may comprise Zinc, Tin, Magnesium, Copper, Iodide, lithium, aluminum, phosphorus, sulfur, calcium, chromium, manganese, iron, cobalt, nickel, arsenic, strontium, cadmium, tin, iodine, barium, mercury, lead, bismuth, molybdenum, or any combination thereof. In some embodiments, the addition of C-reactive protein immunohistochemistry fluorescence may provide temporal fluctuations of inflammation to complement the diagnostic power of Raman Spectroscopy.

The disease or disorder may comprise autism spectrum disorder (ASD), attention deficit/hyperactivity disorder (ADHD), amyotrophic lateral sclerosis (ALS), schizophrenia, irritable bowel disease (IBD), pediatric kidney disease, kidney transplant rejection, pediatric cancer or any combination thereof. In some examples, the disease or disorder comprises the ASD. In some examples, the subject is a human. In some examples, the subject is an adult. In some examples, the subject is between the ages of about 12 and about 5 years old. In some examples, the subject is less than about 12, 11, 10, 9, 8, 7, 5, 4, 3, 2, or 1 year(s) old. In some examples, the subject is at least about 1, 2, 3, 4, 5, 6, 7, 8, 9, 10, 11, or 12 year(s) old. In some examples, at least a portion of the temporal Raman profile corresponds to a prenatal period of the subject.

In some examples, predicting a subject's diagnostic status with respect to a disease or disorder comprises processing the spatial map using a trained model. In some examples, the processing comprises extracting features from the spatial map (e.g., by recurrence quantification analysis), and analyzing the features using the trained model. In some examples, the processing comprises computational analysis of temporal dynamics derived from the spatial map, e.g., by application of dimensionality reduction techniques, including independent component analysis (ICA) and/or principal component analysis (PCA), followed by the subsequent application of recurrence quantification analysis (RQA) to extract computational features descriptive of the dimensions derived from ICA/PCA.

In some examples, the trained model comprises a plurality of parameters, where the term "parameter" refers to any coefficient or, similarly, any value of an internal or external element (e.g., a weight and/or a hyperparameter) in the model (e.g., where the model is a regressor or a classifier) that can affect (e.g., modify, tailor, and/or adjust) one or more inputs, outputs, and/or functions in the model. For example, in some examples, a parameter of a model refers to any coefficient, weight, and/or hyperparameter that can be used to control, modify, tailor, and/or adjust the behavior, learning, and/or performance of the model. In some instances, a parameter is used to increase or decrease the influence of an input (e.g., a feature) to a model. As a nonlimiting example, a parameter is used to increase or decrease the influence of a node (e.g., of a neural network), where the node includes one or more activation functions. Assignment of parameters to specific inputs, outputs, and/or functions of a model is not limited to any one paradigm for a given model but can be used in any suitable model for a desired performance. In some examples, a parameter has a fixed value. In some examples, a value of a parameter is manually and/or automatically adjustable. In some examples, a value of a parameter is modified by a validation and/or training process for a model (e.g., by error minimization and/or back propagation methods). In some examples, a model of the present disclosure includes a plurality of parameters. In some examples, the plurality of parameters associated with a model (e.g., an untrained, partially trained, or fully trained model) is n parameters, where: $n \geq 2$; $n \geq 5$; $n \geq 10$; $n \geq 25$; $n \geq 40$; $n \geq 50$; $n \geq 75$; $n \geq 100$; $n \geq 125$; $n \geq 150$; $n \geq 200$; $n \geq 225$; $n \geq 250$; $n \geq 350$; $n \geq 500$; $n \geq 600$; $n \geq 750$; $n \geq 1,000$; $n \geq 2,000$; $n \geq 4,000$; $n \geq 5,000$; $n \geq 7,500$; $n \geq 10,000$; $n \geq 20,000$; $n \geq 40,000$; $n \geq 75,000$; $n \geq 100,000$; $n \geq 200,000$; $n \geq 500,000$, $n \geq 1 \times 10^6$, $n \geq 5 \times 10^6$, or $n \geq 1 \times 10^7$. In some examples n is between 10,000 and $1 \times 10^7$, between 100,000 and $5 \times 10^6$, or between 500,000 and $1 \times 10^6$.

The trained model may be selected from the group consisting of: a neural network algorithm, a support vector machine algorithm, a decision tree algorithm, an unsupervised clustering algorithm, a supervised clustering algorithm, a regression algorithm, a gradient-boosting algorithm (e.g., a gradient-boosting implementation of a machine learning algorithm such as gradient-boosted decision trees) and any combination thereof. In some examples, the trained model comprises a gradient-boosted ensemble model. In some examples, the trained model is configured to process one or more features selected from the group consisting of recurrence rates, determinism, mean diagonal length, maximum diagonal length, divergence, Shannon entropy in diagonal length, trend in recurrences, laminarity, trapping time, maximum vertical line length, Shannon entropy in vertical line lengths, mean recurrence time, Shannon entropy in recurrence times, number of the most probable recurrences, and/or any combination thereof. In some examples, the trained model is configured to process two or more features selected from the group consisting of recurrence rates, determinism, mean diagonal length, maximum diagonal length, divergence, Shannon entropy in diagonal length, trend in recurrences, laminarity, trapping time, maximum vertical line length, Shannon entropy in vertical line lengths, mean recurrence time, Shannon entropy in recurrence times, number of the most probable recurrences, and/or any combination thereof. In some examples, the method further comprises predicting a subject's diagnostic status with respect to the disease or disorder with a sensitivity of at least about 80%. In some examples, the method further comprises predicting a subject's diagnostic status with respect to the disease or disorder with a sensitivity of up to about 80%. In some examples, the method further comprises predicting a subject's diagnostic status with respect to the disease or disorder with a specificity of at least about 80%. In some examples, the method further comprises predicting a subject's diagnostic status with respect to the disease or disorder with a specificity of up to about 80%. In some examples, the method further comprises predicting a subject's diagnostic status with respect to the disease or disorder with a positive predictive value of at least about 80%. In some examples, the method further comprises predicting a subject's diagnostic status with respect to the disease or disorder with a positive predictive value of up to about 80%. In some examples, the method further comprises predicting a subject's diagnostic status with respect to the disease or disorder with a negative predictive value of at least about 80%. In some examples, the method further comprises predicting a subject's diagnostic status with respect to the disease or disorder with a negative predictive value of up to about 80%. In some examples, the method further comprises predicting a subject's diagnostic status with respect to the disease or disorder with an Area Under the Receiver Operating Characteristic (AUROC) of at least about 0.80.

In another aspect, the present disclosure provides a device comprising one or more processors, and memory storing one or more programs for execution by the one or more processors, the one or more programs comprising instructions for: (a) sampling each respective position in a plurality of positions along a reference line on a biological sample of a subject associated with a Raman signature of the subject, thereby obtaining a plurality of Raman spectra, each Raman spectrum in the plurality of Raman spectra corresponding to a different position in the plurality of positions, and each position in the plurality of positions representing a different period of growth of the biological sample associated with the Raman signature; (b) analyzing each of the plurality of Raman spectra across a reference line on the biological sample thereby obtaining a first dataset; (c) deriving a respective second dataset from the corresponding plurality of the Raman spectra measurements, each respective feature in the corresponding set of features being determined by a sequential variation in the Raman spectra; and (d) processing the features using a trained model to predict a subject's diagnostic status with respect to disease or disorder associated with the Raman signature. In some examples, the respective second dataset is derived by applying recurrence quantification analysis or related methods to the corresponding plurality of Raman spectra measurements. In some examples, the analyzing of the Raman spectra comprises cosmic ray removal, background correction, normalization, peak fitting, or any combination thereof.

In another aspect, the present disclosure provides a non-transitory computer readable storage medium and one or more computer programs embedded therein for classification, the one or more computer programs comprising instructions which, when executed by a computer system, cause the computer system to perform a method comprising: (a) sampling each respective position in a plurality of positions along a reference line on a biological sample of a subject associated with a Raman signature of the subject, thereby obtaining a plurality of Raman spectra, each Raman spectrum in the plurality of Raman spectra corresponding to a different position in the plurality of positions, and each position in the plurality of positions representing a different period of growth of the biological sample associated with the Raman signature; (b) analyzing each of the plurality of Raman spectra across a reference line on the biological sample thereby obtaining a first dataset; (c) deriving a respective second dataset from the corresponding plurality of the Raman spectra measurements, each respective feature in the corresponding set of features being determined by a sequential variation in the Raman spectra; and (d) processing the features using a trained model to predict a subject's diagnostic status with respect to disease or disorder associated with the Raman signature. In some examples, the respective second dataset is derived by applying recurrence quantification analysis or related methods to the corresponding plurality of Raman spectra measurements. In some examples, the analyzing of the Raman spectra comprises cosmic ray removal, background correction, normalization, peak fitting, or any combination thereof.

Not claimed but disclosed for illustrative purposes is a method for training a model, comprising: at a computer system having one or more processors, and memory storing one or more programs for execution by the one or more processors: (a) for each respective training subject in a plurality of training subjects, wherein a first subset of training subjects in the plurality of training subjects have a first diagnostic status corresponding to having a first biological condition associated with a Raman signature and a second subset of training subjects in the plurality of training subjects have a second diagnostic status corresponding to not having the first biological condition associated with the Raman signature: (i) sampling each respective position in a plurality of positions along a reference line on a biological sample of the subject associated with the Raman signature of the subject, thereby obtaining a plurality of Raman spectra, each Raman spectrum in the plurality of Raman spectra corresponding to a different position in the plurality of positions, and each position in the plurality of positions represent a different period of growth of the biological sample of the subject associated with the Raman signature; (ii) analyzing each Raman spectrum across reference line on biological sample thereby obtaining a first dataset; and (iii) deriving a respective second dataset from the corresponding plurality of Raman spectra, each respective feature in the corresponding set of features being determined by a sequential variation in Raman spectra; and (b) training an untrained or partially untrained model with (i) the corresponding set of features of each respective second dataset of each training subject in the plurality of training subjects and (ii) the corresponding diagnostic status of each training subject in the plurality of training subjects, selected from among the first diagnostic status and the second diagnostic status, thereby obtaining a trained model that provides an indication as to whether a test subject has the first biological condition associated with the Raman signature based on values for features in a set of features acquired from a biological sample associated with the Raman signature of the test subject. In some examples, the respective second dataset is derived by applying recurrence quantification analysis or related methods to the corresponding plurality of Raman spectra measurements. In some examples, the analyzing of the Raman spectra comprises cosmic ray removal, background correction, normalization, peak fitting, or any combination thereof.

In some examples, the trained model is a neural network algorithm, a support vector machine algorithm, a decision tree algorithm, an unsupervised clustering model algorithm, a supervised clustering model algorithm, a regression model, or a gradient-boosting algorithm (e.g., a gradient-boosting implementation of a machine learning algorithm such as gradient-boosted decision trees). In some examples, the trained model is a multinomial classifier. In some examples, the trained model is a binomial classifier. In some examples, the first biological condition is selected from the group consisting of autism spectrum disorder (ASD), attention-deficit/hyperactivity disorder (ADHD), amyotrophic lateral sclerosis (ALS), schizophrenia, irritable bowel disease (IBD), pediatric kidney disease, kidney transplant rejection, and pediatric cancer.

In some examples, evaluating the test subject for the first biological condition associated with a Raman signature further includes discriminating between a presence of the first biological condition associated with the Raman signature and an absence of the first biological condition associated with the Raman signature. In some examples, evaluating the test subject for the first biological condition associated with the Raman signature further includes discriminating between the first biological condition associated with the Raman signature and a second biological condition associated with the Raman signature distinct from the first biological condition associated with the Raman signature. In some examples, the first biological condition is autism spectrum disorder and the second biological condition is neurotypical development; that is, the absence of a neurodevelopmental disorder. In some examples, the first biological condition is autism spectrum disorder and the second biological condition is attention-deficit/hyperactivity disorder. In some examples, the test subject is a human. In some examples, the test subject is an adult. In some examples, the human is between the ages of about 12 and about 5 years old. In some examples, the subject is less than about 12, 11, 10, 9, 8, 7, 5, 4, 3, 2, or 1 year(s) old. In some examples, the subject is at least about 1, 2, 3, 4, 5, 6, 7, 8, 9, 10, 11, or 12 year(s) old. In some examples, at least a portion of the temporal profile of the Raman profile corresponds to a prenatal period of the subject.

The corresponding biological sample associated with the Raman signature of the respective training subject may be selected from the group consisting of a hair shaft, a tooth, and a nail. The corresponding biological sample associated with the Raman signature of the respective training subject may be the hair shaft, and wherein the reference line corresponds to a longitudinal direction of the hair shaft. In some examples, the corresponding biological sample associated with the Raman signature of the respective training subject is the tooth, and wherein the reference line corresponds to a direction across the growth bands, including the neonatal line of the tooth. In some examples, the corresponding plurality of positions is sequenced such that a first position in the corresponding plurality of positions along the corresponding biological sample of the respective training subject corresponds to a position closest to a tip of the corresponding biological sample of the respective training subject. In some examples, each trace in the corresponding plurality of Raman spectra measurements includes a plurality of data points, each data point being an instance of the respective position in the plurality of positions. In some examples, the corresponding set of features is selected from the group consisting of laminarity, entropy, trapping time (TT), mean diagonal length (MDL), recurrence time (RT), Vmax, determinism, Lmax, and any combination thereof. In some examples, the corresponding plurality of positions includes at least 1000, 1500, 2000, 2500, 3000, 3500, 4000, 4500, 5000, 5500, 6000, 6500, 7000, 7500, 8000, 8500, 9000, 9500, 10000, or more than 10000 positions.

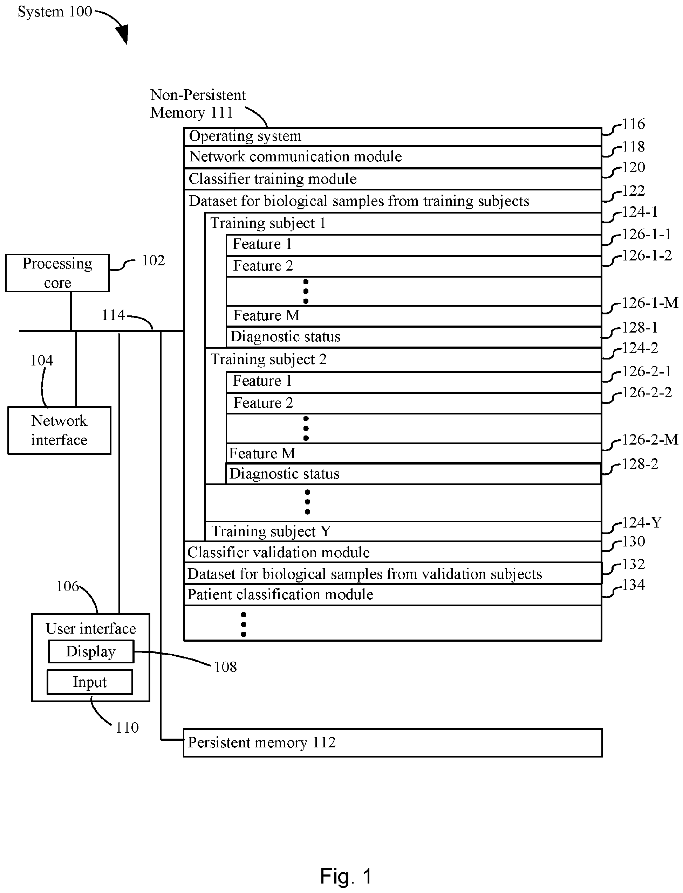

Details of an exemplary system are described in conjunction with FIG. 1, which shows an example of a block diagram of a computing device 100 of the present disclosure. The device 100 in some implementations includes one or more processing units CPU(s) 102 (also referred to as processors), one or more network interfaces 104, a user interface 106, a non-persistent memory 111, a persistent memory 112, and one or more communication buses 114 for interconnecting these components. The one or more communication buses 114 optionally include circuitry (sometimes called a chipset) that interconnects and controls communications between system components. The non-persistent memory 111 typically includes high-speed random access memory, such as DRAM, SRAM, DDR RAM, ROM, EEPROM, flash memory, whereas the persistent memory 112 typically includes CD-ROM, digital versatile disks (DVD) or other optical storage, magnetic cassettes, magnetic tape, magnetic disk storage or other magnetic storage devices, magnetic disk storage devices, optical disk storage devices, flash memory devices, or other non-volatile solid state storage devices. The persistent memory 112 optionally includes one or more storage devices remotely located from the CPU(s) 102. The persistent memory 112, and the non-volatile memory device(s) within the non-persistent memory 112, comprise non-transitory computer readable storage medium. In some implementations, the non-persistent memory 111 or alternatively the non-transitory computer readable storage medium stores the following programs, modules and data structures, or a subset thereof, sometimes in conjunction with the persistent memory 112: an optional operating system 116, which includes procedures for handling various basic system services and for performing hardware dependent tasks; an optional network communication module (or instructions) 118 for connecting the system 100 with other devices and/or a communication network 104; an optional classifier training module 120 for training models (e.g., classifiers, regressors, etc.) for evaluating a subject for a biological condition; an optional data store 122 for datasets for biological samples from training subjects, including feature data for one or more training subjects 124, where the feature data includes a parameter associated with each of features 126, and diagnostic status 128 (e.g., an indication that a respective training subject has been diagnosed with a biological condition or has not been diagnosed with a biological condition); an optional classifier validation module 130 for validating models that distinguish the a biological condition; an optional data store 132 for datasets for biological samples from validation subjects; and an optional patient classification module 134 for classifying a subject as having a biological condition, e.g., as trained using classifier training module 120.

In various implementations, one or more of the above identified elements are stored in one or more of the previously mentioned memory devices, and correspond to a set of instructions for performing a function described above. The above identified modules, data, or programs (e.g., sets of instructions) need not be implemented as separate software programs, procedures, datasets, or modules, and thus various subsets of these modules and data may be combined or otherwise re-arranged in various implementations. In some implementations, the non-persistent memory 111 optionally stores a subset of the modules and data structures identified above. Furthermore, the memory stores additional modules and data structures not described above. One or more of the above identified elements may be stored in a computer system, other than that of visualization system 100, that is addressable by visualization system 100 so that visualization system 100 may retrieve all or a portion of such data when needed.

The system 100 may be connected to, or includes, one or more analytical devices for performing chemical analyzes. For example, the optional network communication module (or instructions) 118 is configured to connect the system 100 with the one or more analytical devices, e.g., via the communication network 104. In some examples, the one or more analytical devices include a laser ablation-inductively coupled plasma-mass spectrometer (LA-ICP-MS), a fluorescence image sensor, or a Raman spectrometer.

Although **FIG.** 1 depicts a "system 100," the figure is intended more as functional description of the various features which may be present in computer systems than as a structural schematic of the implementations described herein. In practice, and as recognized by those of ordinary skill in the art, items shown separately may be combined and some items may be separated. Moreover, although FIG. 1 depicts certain data and modules in non-persistent memory 111, some or all of these data and modules may be in persistent memory 112.

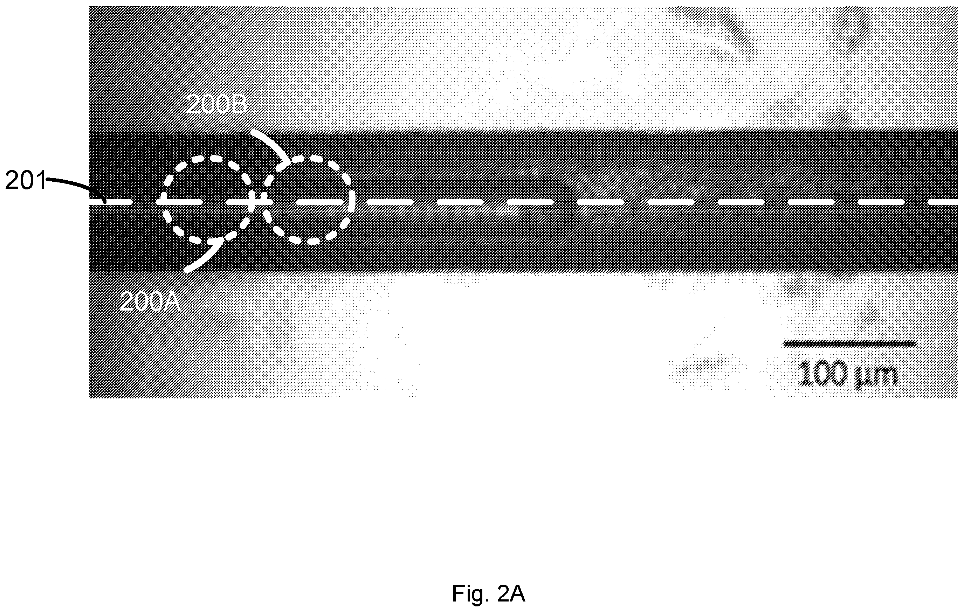

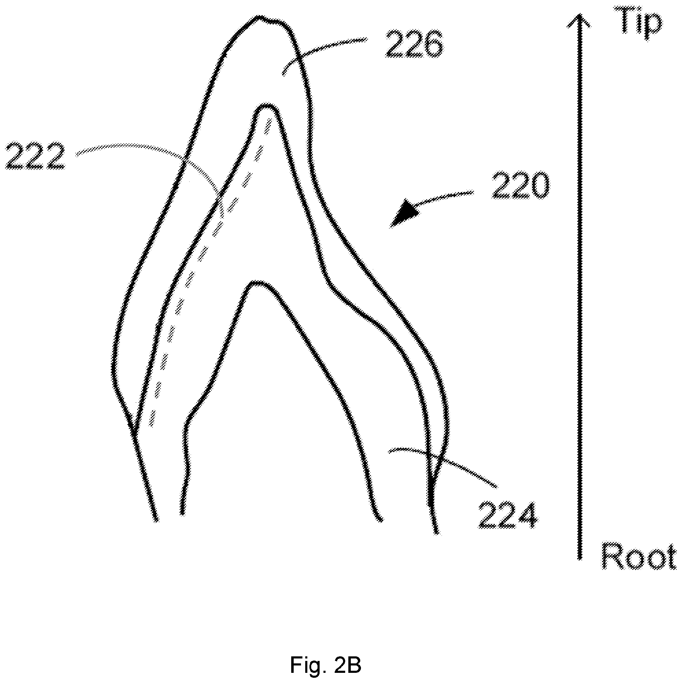

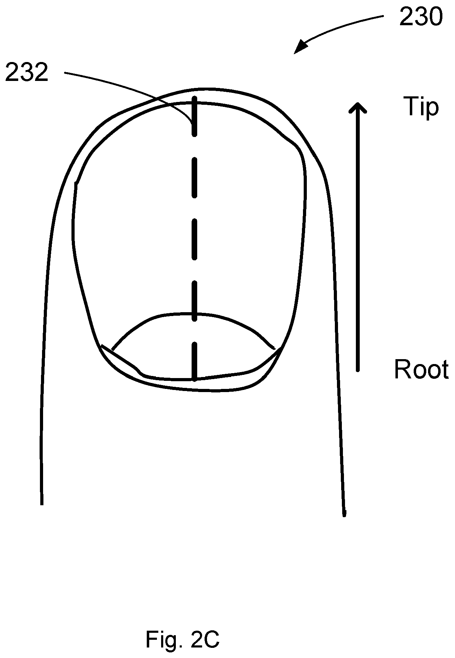

A method of the present disclosure comprises obtaining a biological sample (e.g., a strand of hair including a hair shaft). The subject may be a human. In some examples, the subject is a child aged equal to or below 12 years (e.g., the child is aged equal to or below 5 years, 4 years, 3 years, 2 years, 1 year, 9 months, 6 months, 3 months, or 1 month). In some examples, the child is between the ages of about 12 and about 5 years old. In some examples, the subject is less than about 12, 11, 10, 9, 8, 7, 5, 4, 3, 2, or 1 year(s) old. In some examples, the subject is at least about 1, 2, 3, 4, 5, 6, 7, 8, 9, 10, 11, or 12 year(s) old. In some examples, the subject is an adult. In some examples, the subject is an adult. FIG. 2A shows an example of a hair sample of a subject including a hair shaft. The hair sample may be simply cut from the subject (e.g., with help of scissors). The method of obtaining the hair sample may be non-invasive. The obtained hair sample may have a minimum length of 1 cm (e.g., the hair sample is 1 cm, 2 cm, 3 cm, 4 cm, or 5 cm long). The hair sample may include any portion of a hair (e.g., a tip or a portion between the tip and a follicle). In particular, there is no special requirement for the hair sample to include the hair follicle. FIG. 2B shows an example of a tooth sample of a subject. FIG. 2C shows an example of a nail sample of a subject. In instances of a nail or a hair, obtaining a biological sample may refer to positioning the subject such that the nail or the hair may be sampled. The nail sample may comprise a whole nail or a nail clipping.

In some examples, the obtained biological sample is pre-processed, such as being pre-treated by washing the biological sample with one or more solvents and/or surfactants and drying. In an instance that the biological sample is a hair, the hair sample may be washed in a solution of TRITON X-100® and ultrapure metal free water (e.g., MILLI-Q® water) and dried overnight in an oven (e.g., at 60 degrees Celsius). The pre-treatment may further include preparing the hair shaft for a measurement by placing the hair shaft on a glass slide (e.g., a microscopic glass slide) with an adhesive film (e.g., a double-sided tape). The hair shaft may be positioned such that the hair shaft is substantially straight. The glass slide with the hair shaft may be placed into or in the vicinity of a measurement system (e.g., a laser ablation-inductively coupled plasma-mass spectrometer (LA-ICP-MS), a fluorescence image sensor, or a Raman spectrometer) for performing analysis. In an instance that the biological sample is a tooth or a nail, a surface of the biological sample may be cleaned (e.g., by surfactant, water, or one or more solvents). The sample may be sectioned and then placed into or in the vicinity of a measurement system (e.g., a laser ablation-inductively coupled plasma-mass spectrometer (LA-ICP-MS), a fluorescence image sensor, or a Raman spectrometer) for performing analysis.

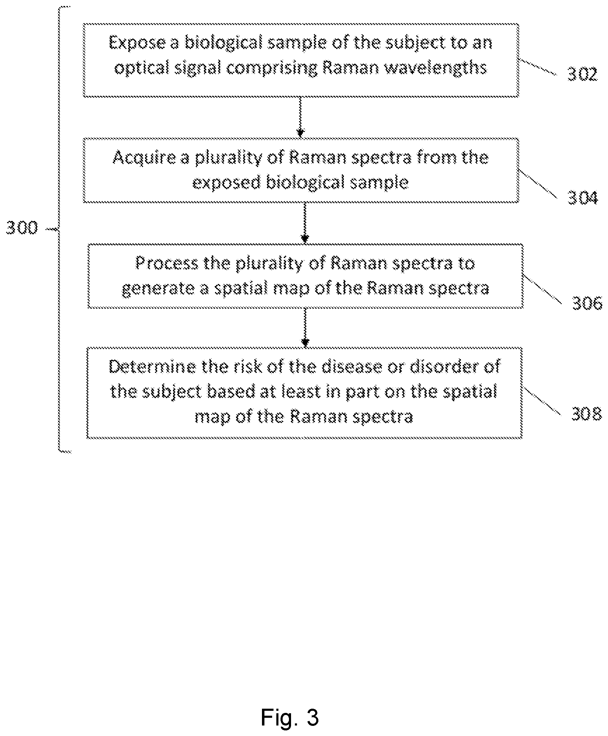

FIG. 3 shows a flow chart of a method 300 for evaluating a subject for a biological condition, such as a method for predicting a subject's diagnostic status with respect to a disease or disorder. The method 300 may comprise exposing a biological sample of the subject to a light source (as in operation 302). In some cases, the light source may comprise a laser. In some examples, the analyzing determines temporal dynamics of underlying biological processes. In some examples, the analyzing comprises reducing a dimensionality of the plurality of Raman spectra (e.g., by independent components analysis) prior to the processing. In some examples, the optical signal is generated by a light source (e.g., a laser). The biological sample may comprise a tooth sample, a hair sample, or a nail sample. Next, the method 300 may comprise acquiring a plurality of Raman spectra from the exposed biological sample (as in operation 304). Next, the method 300 may comprise processing the plurality of Raman spectra to generate a spatial map of the plurality of Raman spectra (as in operation 306). Next, the method 300 may comprise predicting a subject's diagnostic status with respect to a disease or disorder based at least in part on the spatial map of the plurality of Raman spectra (as in operation 308).

In some examples, the plurality of Raman spectra are acquired using a Raman spectroscopy microscope, including a 50X air coupled objective or a 63X water immersion coupled objection. In some examples, the laser comprises a wavelength of about 785 nm, or a wavelength of about 532 nm. In some examples, the acquiring is performed using an integration time of about 0.2 seconds to about 0.3 seconds. In some examples, the acquiring comprises moving the biological sample with a step size of about 2 microns to about 5 microns, subsequent to acquiring a Raman spectrum of the plurality of Raman spectra.

In some examples, the analyzing comprises generating a temporal Raman profile based at least in part on the Raman spectra acquired, and analyzing the temporal profile of variability in the Raman spectra. In some examples, at least a portion of the temporal Raman profile corresponds to a prenatal period of the subject.

Measurement data may be collected from the biological sample sequentially at a plurality of positions along the biological sample. In some examples, the corresponding plurality of positions includes at least 1000, 1500, 2000, 2500, 3000, 3500, 4000, 4500, 5000, 5500, 6000, 6500, 7000, 7500, 8000, 8500, 9000, 9500, 10000, or more than 10000 positions. In some examples, the respective positions are adjacent to each other. By this method, each area corresponding to a distinct position on the biological sample may be thereby associated with a dynamic (e.g., time-varying) abundance measurement. In some examples, the respective positions are separated by a predefined distance. In some examples, the sampling is performed along the reference line of the biological sample starting from a respective position nearest to the tip of the biological sample such as hair sample (e.g., at a position that corresponds to the youngest age of the subject). In general, the sampling can be performed starting from a respective position nearest to the tip or the root, as long as the direction of the sampling is known, and an appropriate trained model is used for the analyses.

The sampling may produce sets of data points. Each set of data points may correspond to a measurement (e.g., an abundance or concentration) of a substance that is indicative of a dynamic biological response measured at a plurality of positions along the biological sample. Each position on the reference line of the biological sample may correspond to a specific time of growth of the biological sample. In some examples, in an instance of the hair shaft, each position corresponds to approximately 20 min period of hair growth (e.g., the period of hair growth calculated using a 5-micrometer laser step size and an average rate of hair growth 1 cm per month). By correlating the plurality of positions along the reference line of the biological sample to corresponding time periods of the growth, a first dataset including a plurality of traces is obtained. Each trace includes a time-dependent abundance of a measurement (e.g., an abundance or concentration) of a substance that is indicative of a dynamic biological response measured from the biological sample. For example, the distance between positions may correspond to an estimated growth of the biological sample (e.g., biological time). For example, abundance may be measured for a hair sample along a 1.2 cm distance, which corresponds to a biological time of approximately 35 days. The biological time may be estimated by using an average rate of hair growth (e.g., 1 cm per month).

Data analysis of the Raman spectra may comprise of cosmic ray removal, background correction, spectral normalization, peak fitting, or any combination therein.

Data analysis may be performed on the traces corresponding to a time-dependent abundance (e.g., a time-dependent concentration) of a substance that is indicative of a dynamic biological response measured from the biological sample. This may comprise customized operations to clean the data (e.g., smoothening the data over a time span, and/or removing data points that are higher or lower than a predetermined threshold). In some examples, the data analysis includes removing, from the traces, data points that have a mean absolute difference between adjacent data points that is at least one, two, or three times a standard deviation of the mean absolute difference between adjacent points.

In some examples, the data analysis further includes a dimension-reduction step, whereby the high-dimensional array of Raman spectra are decomposed into a lower dimensional array of derived time-varying components. Methods for dimensionality-reduction include independent component analysis (ICA), principal component analysis (PCA), non-negative matrix factorization (NNMF), and related unsupervised and supervised methods.

In some examples, the data analysis further includes performing recurrence quantification analysis (RQA) on the time-dependent traces, or on components derived from dimensionality-reduction techniques (ICA/PCA) applied to the time-dependent traces, to obtain a set of features that describe dynamical periodical characteristics of the traces. RQA measures variability in the time-dependent traces or components derived from the time-dependent traces. RQA involves the estimation of features that describe periodic properties in a given waveform, which include the recurrence rates, determinism, mean diagonal length, maximum diagonal length, divergence, Shannon entropy in diagonal length, trend in recurrences, laminarity, trapping time, maximum vertical line length, Shannon entropy in vertical line lengths, mean recurrence time, Shannon entropy in recurrence times, number of the most probable recurrences, and/or any combination thereof. Methods and features of RQA are described, for example, by Webber et al. in "Simpler Methods Do It Better: Success of Recurrence Quantification Analysis as a General Purpose Data Analysis Tool," Physics Letters A 373, 3753-3756 (2009) and by Marwan et al. in "Recurrence Plots for the Analysis of Complex Systems," Physics Reports 438, 237-239 (2007). In some examples, the time-dependent traces are analyzed by using other analytical methods, such as Fourier Transformations, Wavelet Analysis, and Cosinor analysis. Such techniques can be applied to derive similar metrics, including spectral analysis of frequency components and their associated power. These metrics and associated derivative measures may be used in place of the features derived from RQA to analyze the time-dependent traces obtained from biological samples for purposes of predictive classification.

The RQA includes construction of recurrence plots that visualize and analyze dynamical temporal structures in respective obtained traces. Such recurrence plots may illustrate phasic processes in sequential measurements by plotting a given sequence against a time-lagged derivation of that sequence. From the one dimensional trace measured from the hair shaft, additional dimensions are computationally derived to embed the trace in a higher dimensional space referred to as a phase portrait, where t refers to the values of the original trace, and dimensions (t+τ) and (t+2τ) are derived from lagging the original time series by interval τ. Subsequent analyses are then undertaken on the embedded phase portrait to construct recurrence plots and recurrence quantification analysis. A recurrence quantification plot may be derived from the phase portrait through the application of a threshold function to each point in the phase portrait; on the corresponding recurrence plot, consisting of a square binary matrix, typically represented as white or black space, a given point is assigned a value of 1 at each temporal interval wherein another point in the phase-portrait shares the spatial limits of the assigned threshold boundary. The RQA method is applied to the recurrence plot to examine the interval of delay between states in a given system, with a black point reflecting the temporal interval when a system revisits the same state. Periodic processes, where a system successively reiterates a given pattern of states, will manifest in a recurrence plot as diagonal black lines, whereas periods of stability will manifest as square structures, spurious repetitions as black dots, and, unique events as white space.

In some examples, the recurrence plots are constructed for traces of a single substance or a combination of two substances (e.g., in order to visualize an interactive periodic pattern of two substances; this can be referred to as cross-recurrence quantification analysis, or joint-recurrence quantification analysis). In some examples, the recurrence plots are constructed for a combination of three or more substances.

In some examples, the data analysis includes analyzing the recurrence plots to obtain a set of features associated with the recurrence plots. The features, which interchangeably can be termed "rhythmicity features," or "dynamic features," provide a quantitative measure describing the periodicity, predictability, and transitivity present in the plurality of traces. The features are selected from a set including recurrence rates, determinism, mean diagonal length, maximum diagonal length, divergence, Shannon entropy in diagonal length, trend in recurrences, laminarity, trapping time, maximum vertical line length, Shannon entropy in vertical line lengths, mean recurrence time, Shannon entropy in recurrence times, number of the most probable recurrences, and/or any combination thereof.

In some examples, the data analysis further includes inputting the obtained set of features to a trained models. In some examples, the trained model includes a predictive computational algorithm to obtain a probability for the subject having a biological condition. In some examples, the predictive computational algorithm performs the following calculation:

$$p(subject) = \frac{1}{1 + e^{-(\alpha + \beta_1 x_1 + \cdots + \beta_k x_k)}}$$

where $p(subject)$ is the probability that the subject has the first biological condition, e is Euler's number, $\alpha$ is a calculated parameter associated with the probability that the subject has the biological condition when $\beta_1 x_1 + ... + \beta_k x_k$ equals to zero, $x_1, ..., x_k$ corresponds to a value derived for each feature in the set of features, the set of features including features from 1 through $k$, and $\beta_1, ..., \beta_k$ corresponds to a weight parameter associated with each feature in the set of features including features from 1 through k.

The weight parameters $\beta_1, ..., \beta_k$ may be defined based on model training. The probability *p(subject)* may be provided as a number ranging from 0 to 1, where 1 corresponds to a 100% probability that the subject has a biological condition.

In some examples, the data analysis includes applying a threshold to the obtained probability *p(subject)*. If the obtained probability *p(subject)* is above the predetermined threshold, the subject is evaluated as having the biological condition. If the obtained probability is below the threshold, the subject is evaluated as not having the biological condition. In some examples, the threshold is between about 0.3 and 0.6 (e.g., the predetermined threshold is about 0.3, 0.35, 0.4, 0.45, 0.5, 0.55, or 0.6). The value assigned for a probabilistic threshold may be predetermined, or estimated during the training of the model through the use of receiver-operating-characteristic (ROC) charts, with the optimal threshold used corresponding to the value which yields the maximum area-under-the-curve (ROC-AUC). In some examples, the obtained probability is expressed in terms of associated odds (e.g., odds ratio (OR), which may be derived from a probability such that OR=p/(1-p)). For example, the evaluation includes evaluating odds that the subject has the biological condition.

In some examples, the data analysis includes discriminating a first biological condition from an alternative condition, e.g., a second, biological condition. In some examples, the alternative condition is associated with no known condition (e.g., a neurotypical condition (NT)). In some examples, the first biological condition is associated with autism spectrum disorder (ASD) and the alternative condition is associated with an attention-deficit/hyperactivity disorder (ADHD). In some examples, the alternative condition is any other neurodevelopmental condition, or a comorbid diagnosis for two neurodevelopmental conditions. Therefore, the data analysis may be capable of discriminating between two neurodevelopmental conditions (e.g., between autism spectrum disorder and ADHD, or between ASD and co-morbid (CM) cases diagnosed for both ASD and ADHD).

Health care providers, such as physicians and treating teams of a patient may have access to patient data (e.g., dynamic biological response data or other health data), and/or predictions or assessments generated from such data. Based on the data analysis results, health care providers may determine clinical decisions or outcomes.

For example, a physician may instruct that patient undergo one or more clinical tests at the hospital or other clinical site, based at least in part on a predicted disease or disorder in the subject. These instructions may be provided when a certain pre-determined criterion is met (e.g., a minimum threshold for a likelihood of the disease or disorder).

Such a minimum threshold may be, for example, at least about a 5% likelihood, at least about a 10% likelihood, at least about a 20% likelihood, at least about a 25% likelihood, at least about a 30% likelihood, at least about a 35% likelihood, at least about a 40% likelihood, at least about a 45% likelihood, at least about a 50% likelihood, at least about a 55% likelihood, at least about a 60% likelihood, at least about a 65% likelihood, at least about a 70% likelihood, at least about a 75% likelihood, at least about an 80% likelihood, at least about a 85% likelihood, at least about a 90% likelihood, at least about a 95% likelihood, at least about a 96% likelihood, at least about a 97% likelihood, at least about a 98% likelihood, or at least about a 99% likelihood.

As another example, a physician may prescribe a therapeutically effective dose of a treatment (e.g., drug), a clinical procedure, or further clinical testing to be administered to the patient based at least in part on a predicted disease or disorder in the subject. For example, the physician may prescribe an anti-inflammatory therapeutic in response to an indication of inflammation in the patient.

## Models

The methods and systems of the present disclosure may utilize or access external capabilities of artificial intelligence techniques to develop signatures for various diseases or disorders. These signatures may be used to accurately predict diseases or disorders (e.g., months or years earlier than with standard of clinical care). Using such a predictive capability, health care providers (e.g., physicians) may be able to make informed, accurate risk-based decisions, thereby improving quality of care and monitoring provided to patients.

The methods and systems of the present disclosure may analyze acquired dynamic biological response data from a subject (patient) to generate a likelihood of the subject having a disease or disorder. For example, the system may apply a trained (e.g., prediction) algorithm to the acquired dynamic biological response data to generate the likelihood of the subject having a disease or disorder. The trained algorithm may comprise an artificial intelligence-based model, such as a machine learning based classifier, configured to process the acquired dynamic biological response data to generate the likelihood of the subject having the disease or disorder. The model be trained using clinical datasets from one or more cohorts of patients, e.g., using clinical health data and/or dynamic biological response data of the patients as inputs and known clinical health outcomes (e.g., disease or disorder) of the patients as outputs to the model.

The model may comprise one or more machine learning algorithms. Examples of machine learning algorithms may include a support vector machine (SVM), a naive Bayes classification, a random forest, a neural network (such as a deep neural network (DNN), a recurrent neural network (RNN), a deep RNN, a long short-term memory (LSTM) recurrent neural network (RNN), a gated recurrent unit (GRU), or other supervised learning algorithm or unsupervised machine learning, statistical, or deep learning algorithm for classification and regression. The model may likewise involve the estimation of ensemble models, comprised of multiple predictive models, and utilize techniques such as gradient boosting, for example in the construction of gradient-boosting decision trees. The model may be trained using one or more training datasets corresponding to patient data.

Training datasets may be generated from, for example, one or more cohorts of patients having common clinical characteristics (features) and clinical outcomes (labels). Training datasets may comprise a set of features and labels corresponding to the features. Features may correspond to algorithm inputs comprising dynamic biological response data, patient demographic information derived from electronic medical records (EMR), and medical observations. Features may comprise clinical characteristics such as, for example, certain ranges or categories of dynamic biological response data. Features may comprise patient information such as patient age, patient medical history, other medical conditions, current or past medications, and time since the last observation. For example, a set of features collected from a given patient at a given time point may collectively serve as a signature, which may be indicative of a health state or status of the patient at the given time point.

For example, ranges of dynamic biological response data and other health measurements may be expressed as a plurality of disjoint continuous ranges of continuous measurement values, and categories of dynamic biological response data and other health measurements may be expressed as a plurality of disjoint sets of measurement values (e.g., {"high", "low"}, {"high", "normal"}, {"low", "normal"}, {"high", "borderline high", "normal", "low"}, etc.). Clinical characteristics may also include clinical labels indicating the patient's health history, such as a diagnosis of a disease or disorder, a previous administration of a clinical treatment (e.g., a drug, a surgical treatment, chemotherapy, radiotherapy, immunotherapy, etc.), behavioral factors, or other health status (e.g., hypertension or high blood pressure, hyperglycemia or high blood glucose, hypercholesterolemia or high blood cholesterol, history of allergic reaction or other adverse reaction, etc.).

Labels may comprise clinical outcomes such as, for example, a presence, absence, diagnosis, or prognosis of a disease or disorder in the subject (e.g., patient). Clinical outcomes may include a temporal characteristic associated with the presence, absence, diagnosis, or prognosis of the disease or disorder in the patient. For example, temporal characteristics may be indicative of the patient having had an occurrence of the disease or disorder within a certain period of time after a previous clinical outcome (e.g., being discharged from the hospital, being administered a treatment such as medication, undergoing a clinical procedure such as surgical operation, etc.). Such a period of time may be, for example, about 1 hour, about 2 hours, about 3 hours, about 4 hours, about 6 hours, about 8 hours, about 10 hours, about 12 hours, about 14 hours, about 16 hours, about 18 hours, about 20 hours, about 22 hours, about 24 hours, about 2 days, about 3 days, about 4 days, about 5 days, about 6 days, about 7 days, about 10 days, about 2 weeks, about 3 weeks, about 4 weeks, about 1 month, about 2 months, about 3 months, about 4 months, about 6 months, about 8 months, about 10 months, about 1 year, or more than about 1 year.

Input features may be structured by aggregating the data into bins or alternatively using a one-hot encoding. Inputs may also include feature values or vectors derived from the previously mentioned inputs, such as cross-correlations calculated between separate dynamic biological response data or other measurements over a fixed period of time, and the discrete derivative or the finite difference between successive measurements. Such a period of time may be, for example, about 1 hour, about 2 hours, about 3 hours, about 4 hours, about 6 hours, about 8 hours, about 10 hours, about 12 hours, about 14 hours, about 16 hours, about 18 hours, about 20 hours, about 22 hours, about 24 hours, about 2 days, about 3 days, about 4 days, about 5 days, about 6 days, about 7 days, about 10 days, about 2 weeks, about 3 weeks, about 4 weeks, about 1 month, about 2 months, about 3 months, about 4 months, about 6 months, about 8 months, about 10 months, about 1 year, or more than about 1 year.

Training records may be constructed from sequences of observations. Such sequences may comprise a fixed length for ease of data processing. For example, sequences may be zero-padded or selected as independent subsets of a single patient's records.

The model may process the input features to generate output values comprising one or more classifications, one or more predictions, or a combination thereof. For example, such classifications or predictions may include a binary classification of a healthy/normal health state (e.g., absence of a disease or disorder) or an adverse health state (e.g., presence of a disease or disorder), a classification between a group of categorical labels (e.g., 'no disease or disorder', 'apparent disease or disorder', and 'likely disease or disorder'), a likelihood (e.g., relative likelihood or probability) of developing a particular disease or disorder, a score indicative of a presence of disease or disorder, a score indicative of a level of systemic inflammation experienced by the patient, a 'risk factor' for the likelihood of mortality of the patient, a prediction of the time at which the patient is expected to have developed the disease or disorder, and a confidence interval for any numeric predictions. Various machine learning techniques may be cascaded such that the output of a machine learning technique may also be used as input features to subsequent layers or subsections of the model.

In order to train the model (e.g., by determining weights and correlations of the model) to generate real-time classifications or predictions, the model can be trained using datasets. Such datasets may be sufficiently large to generate statistically significant classifications or predictions. For example, datasets may comprise: databases of de-identified data including dynamic biological response data and other measurements, and dynamic biological response data and other measurements from a hospital or other clinical setting.

Datasets may be split into subsets (e.g., discrete or overlapping), such as a training dataset, a development dataset, and a test dataset. For example, a dataset may be split into a training dataset comprising 80% of the dataset, a development dataset comprising 10% of the dataset, and a test dataset comprising 10% of the dataset. The training dataset may comprise about 10%, about 20%, about 30%, about 40%, about 50%, about 60%, about 70%, about 80%, or about 90% of the dataset. The development dataset may comprise about 10%, about 20%, about 30%, about 40%, about 50%, about 60%, about 70%, about 80%, or about 90% of the dataset. The test dataset may comprise about 10%, about 20%, about 30%, about 40%, about 50%, about 60%, about 70%, about 80%, or about 90% of the dataset. Training sets (e.g., training datasets) may be selected by random sampling of a set of data corresponding to one or more patient cohorts to ensure independence of sampling. Alternatively, training sets (e.g., training datasets) may be selected by proportionate sampling of a set of data corresponding to one or more patient cohorts to ensure independence of sampling.

To improve the accuracy of model predictions and reduce overfitting of the model, the datasets may be augmented to increase the number of samples within the training set. For example, data augmentation may comprise rearranging the order of observations in a training record. To accommodate datasets having missing observations, methods to impute missing data may be used, such as forward-filling, back-filling, linear interpolation, and multi-task Gaussian processes. Datasets may be filtered to remove confounding factors. For example, within a database, a subset of patients may be excluded.

The model may comprise one or more neural networks, such as a neural network, a convolutional neural network (CNN), a deep neural network (DNN), a recurrent neural network (RNN), or a deep RNN. The recurrent neural network may comprise units which can be long short-term memory (LSTM) units or gated recurrent units (GRU). For example, the model may comprise an algorithm architecture comprising a neural network with a set of input features such as vital sign and other measurements, patient medical history, and/or patient demographics. Neural network techniques, such as dropout or regularization, may be used during training the model to prevent overfitting. The neural network may comprise a plurality of sub-networks, each of which is configured to generate a classification or prediction of a different type of output information (e.g., which may be combined to form an overall output of the neural network). The machine learning model may alternatively utilize statistical or related algorithms including random forest, classification and regression trees, support vector machines, discriminant analyses, regression techniques, as well as ensemble and gradient-boosted variations thereof.

When the model generates a classification or a prediction of a disease or disorder, a notification (e.g., alert or alarm) may be generated and transmitted to a health care provider, such as a physician, nurse, or other member of the patient's treating team within a hospital. Notifications may be transmitted via an automated phone call, a short message service (SMS) or multimedia message service (MMS) message, an e-mail, or an alert within a dashboard. The notification may comprise output information such as a prediction of a disease or disorder, a likelihood of the predicted disease or disorder, a time until an expected onset of the disease or disorder, a confidence interval of the likelihood or time, or a recommended course of treatment for the disease or disorder.

To validate the performance of the model, different performance metrics may be generated. For example, an area under the receiver-operating curve (AUROC) may be used to determine the diagnostic capability of the model. For example, the model may use classification thresholds which are adjustable, such that specificity and sensitivity are tunable, and the receiver-operating curve (ROC) can be used to identify the different operating points corresponding to different values of specificity and sensitivity.

In some cases, such as when datasets are not sufficiently large, cross-validation may be performed to assess the robustness of a model across different training and testing datasets.

To calculate performance metrics such as sensitivity, specificity, accuracy, positive predictive value (PPV), negative predictive value (NPV), AUPRC, AUROC, or similar, the following definitions may be used. A "false positive" may refer to an outcome in which a positive outcome or result has been incorrectly or prematurely generated (e.g., before the actual onset of, or without any onset of, the disease or disorder). A "true positive" may refer to an outcome in which positive outcome or result has been correctly generated, when the patient has the disease or disorder (e.g., the patient shows symptoms of the disease or disorder, or the patient's record indicates the disease or disorder). A "false negative" may refer to an outcome in which a negative outcome or result has been generated, but the patient has the disease or disorder (e.g., the patient shows symptoms of the disease or disorder, or the patient's record indicates the disease or disorder). A "true negative" may refer to an outcome in which a negative outcome or result has been generated (e.g., before the actual onset of, or without any onset of, the disease or disorder).

The model may be trained until certain pre-determined conditions for accuracy or performance are satisfied, such as having minimum desired values corresponding to diagnostic accuracy measures. For example, the diagnostic accuracy measure may correspond to prediction of a likelihood of occurrence of a disease or disorder in the subject. As another example, the diagnostic accuracy measure may correspond to prediction of a likelihood of deterioration or recurrence of a disease or disorder for which the subject has previously been treated. Examples of diagnostic accuracy measures may include sensitivity, specificity, positive predictive value (PPV), negative predictive value (NPV), accuracy, area under the precision-recall curve (AUPRC), and area under the curve (AUC) of a Receiver Operating Characteristic (ROC) curve (AUROC) corresponding to the diagnostic accuracy of detecting or predicting a disease or disorder.

For example, such a pre-determined condition may be that the sensitivity of predicting the disease or disorder comprises a value of, for example, at least about 50%, at least about 55%, at least about 60%, at least about 65%, at least about 70%, at least about 75%, at least about 80%, at least about 85%, at least about 90%, at least about 95%, at least about 96%, at least about 97%, at least about 98%, or at least about 99%.

As another example, such a pre-determined condition may be that the specificity of predicting the disease or disorder comprises a value of, for example, at least about 50%, at least about 55%, at least about 60%, at least about 65%, at least about 70%, at least about 75%, at least about 80%, at least about 85%, at least about 90%, at least about 95%, at least about 96%, at least about 97%, at least about 98%, or at least about 99%.

As another example, such a pre-determined condition may be that the positive predictive value (PPV) of predicting the disease or disorder comprises a value of, for example, at least about 50%, at least about 55%, at least about 60%, at least about 65%, at least about 70%, at least about 75%, at least about 80%, at least about 85%, at least about 90%, at least about 95%, at least about 96%, at least about 97%, at least about 98%, or at least about 99%.

As another example, such a pre-determined condition may be that the negative predictive value (NPV) of predicting the disease or disorder comprises a value of, for example, at least about 50%, at least about 55%, at least about 60%, at least about 65%, at least about 70%, at least about 75%, at least about 80%, at least about 85%, at least about 90%, at least about 95%, at least about 96%, at least about 97%, at least about 98%, or at least about 99%.

As another example, such a pre-determined condition may be that the area under the curve (AUC) of a Receiver Operating Characteristic (ROC) curve (AUROC) of predicting the disease or disorder comprises a value of at least about 0.50, at least about 0.55, at least about 0.60, at least about 0.65, at least about 0.70, at least about 0.75, at least about 0.80, at least about 0.85, at least about 0.90, at least about 0.95, at least about 0.96, at least about 0.97, at least about 0.98, or at least about 0.99.

As another example, such a pre-determined condition may be that the area under the precision-recall curve (AUPRC) of predicting the disease or disorder comprises a value of at least about 0.10, at least about 0.15, at least about 0.20, at least about 0.25, at least about 0.30, at least about 0.35, at least about 0.40, at least about 0.45, at least about 0.50, at least about 0.55, at least about 0.60, at least about 0.65, at least about 0.70, at least about 0.75, at least about 0.80, at least about 0.85, at least about 0.90, at least about 0.95, at least about 0.96, at least about 0.97, at least about 0.98, or at least about 0.99.

The trained model may be trained or configured to predict the disease or disorder with a sensitivity of at least about 50%, at least about 55%, at least about 60%, at least about 65%, at least about 70%, at least about 75%, at least about 80%, at least about 85%, at least about 90%, at least about 95%, at least about 96%, at least about 97%, at least about 98%, or at least about 99%.

The trained model may be trained or configured to predict the disease or disorder with a specificity of at least about 50%, at least about 55%, at least about 60%, at least about 65%, at least about 70%, at least about 75%, at least about 80%, at least about 85%, at least about 90%, at least about 95%, at least about 96%, at least about 97%, at least about 98%, or at least about 99%.

The trained model may be trained or configured to predict the disease or disorder with a positive predictive value (PPV) of at least about 50%, at least about 55%, at least about 60%, at least about 65%, at least about 70%, at least about 75%, at least about 80%, at least about 85%, at least about 90%, at least about 95%, at least about 96%, at least about 97%, at least about 98%, or at least about 99%.

The trained model may be trained or configured to predict the disease or disorder with a negative predictive value (NPV) of at least about 50%, at least about 55%, at least about 60%, at least about 65%, at least about 70%, at least about 75%, at least about 80%, at least about 85%, at least about 90%, at least about 95%, at least about 96%, at least about 97%, at least about 98%, or at least about 99%.

The trained model may be trained or configured to predict the disease or disorder with an area under the curve (AUC) of a Receiver Operating Characteristic (ROC) curve (AUROC) of at least about 0.50, at least about 0.55, at least about 0.60, at least about 0.65, at least about 0.70, at least about 0.75, at least about 0.80, at least about 0.85, at least about 0.90, at least about 0.95, at least about 0.96, at least about 0.97, at least about 0.98, or at least about 0.99.

The trained model may be trained or configured to predict the disease or disorder with an area under the precision-recall curve (AUPRC) of at least about 0.10, at least about 0.15, at least about 0.20, at least about 0.25, at least about 0.30, at least about 0.35, at least about 0.40, at least about 0.45, at least about 0.50, at least about 0.55, at least about 0.60, at least about 0.65, at least about 0.70, at least about 0.75, at least about 0.80, at least about 0.85, at least about 0.90, at least about 0.95, at least about 0.96, at least about 0.97, at least about 0.98, or at least about 0.99.

The training data sets may be collected from training subjects (e.g., humans). Each training has a diagnostic status indicating that they have either been diagnosed with the biological condition, or have not been diagnosed with the biological condition. In some examples, the training subjects are children aged equal to, or below, 12 years (e.g., equal to or below 5 years, 4 years, 3 years, 2 years, 1 year, 9 months, 6 months, 3 months or 1 month). In some examples, the child is between the ages of about 12 and about 5 years old. In some examples, the subject is less than about 12, 11, 10, 9, 8, 7, 5, 4, 3, 2, or 1 year(s) old. In some examples, the subject is at least about 1, 2, 3, 4, 5, 6, 7, 8, 9, 10, 11, or 12 year(s) old. The following training procedure may be performed for each training subject in a plurality of training subjects.

A plurality of positions of a reference line on a biological sample of the training subject may be sampled, thereby obtaining a plurality of dynamic biological response samples. Each dynamic biological response sample in the corresponding plurality of dynamic biological response samples for a different position in the corresponding plurality of positions, and each position in the corresponding plurality of positions representing a different period of growth of the corresponding biological sample. Next, each respective dynamic biological response sample is analyzed (e.g., using a laser ablation-inductively coupled plasma-mass spectrometer (LA-ICP-MS), a fluorescence image sensor, or a Raman spectrometer) to obtain a plurality of traces. Each trace in the corresponding plurality of traces corresponds to an abundance measurement of a corresponding substance, which are over time collectively determined from the corresponding plurality of dynamic biological response samples.

Next, a respective second dataset may be obtained from the corresponding plurality of traces that includes a corresponding set of features, each respective feature in the corresponding set of features being determined by a variation of abundance of one or more substances in the corresponding plurality of traces as assessed by the application of recurrence quantification analysis or related methods to either the Raman waveform or dimensions derived from the Raman waveform through ICA/PCA or related dimensionality-reduction techniques.

Next, an untrained or partially untrained model may be generated, with (i) the corresponding set of features of each respective second dataset of each training subject in the plurality of training subjects and (ii) the corresponding diagnostic status of each training subject in the plurality of training subjects, selected from among the first diagnostic status and the second diagnostic status, thereby obtaining a trained model. The trained model provides an indication as to whether a test subject has the first biological condition based on values for features in a set of features acquired from a biological sample of the test subject. In some examples, the trained model is a neural network algorithm, a support vector machine algorithm, a decision tree algorithm, an unsupervised clustering algorithm, a supervised clustering algorithm, a regression algorithm, or any combination or variant thereof, particularly including gradient-boosting implementations of the described algorithms, e.g. gradient-boosted decision trees. In some examples, the trained machine learning model utilizes a gradient-boosted ensemble algorithm. In some examples, the trained model is a multinomial or a binomial classifier. In some examples, the trained model can be used to make a binary prediction as to whether a sample was derived from a subject with the first biological condition or not; or, may be multinomial, distinguishing subjects with no diagnosis from those with the first biological condition or a second biological condition, where the second biological condition is distinct from the first biological condition.

In some examples, the model is a neural network or a convolutional neural network. *See,* Vincent et al., 2010, "Stacked denoising autoencoders: Learning useful representations in a deep network with a local denoising criterion," J Mach Learn Res 11, pp. 3371-3408; Larochelle et al., 2009, "Exploring strategies for training deep neural networks," J Mach Learn Res 10, pp. 1-40; and Hassoun, 1995, Fundamentals of Artificial Neural Networks, Massachusetts Institute of Technology.

Independent component analysis (ICA), such as that described herein in the unsupervised dimensionality-reduction of Raman waveforms, is described in Lee, T.-W. (1998): Independent component analysis: Theory and applications, Boston, Mass: Kluwer Academic Publishers, ISBN 0-7923-8261-7, and Hyvärinen, A.; Karhunen, J.; Oja, E. (2001): Independent Component Analysis, New York: Wiley, ISBN 978-0-471-40540-5.

Principal component analysis (PCA), such as that described herein in the unsupervised dimensionality-reduction of Raman waveforms, is described in Jolliffe, I. T. (2002). Principal Component Analysis. Springer Series in Statistics. New York: Springer-Verlag. doi:10.1007/b98835. ISBN 978-0-387-95442-4.

SVMs are described in Cristianini and Shawe-Taylor, 2000, "An Introduction to Support Vector Machines," Cambridge University Press, Cambridge; Boser et al., 1992, "A training algorithm for optimal margin classifiers," in Proceedings of the 5th Annual ACM Workshop on Computational Learning Theory, ACM Press, Pittsburgh, Pa., pp. 142-152; Vapnik, 1998, Statistical Learning Theory, Wiley, New York; Mount, 2001, Bioinformatics: sequence and genome analysis, Cold Spring Harbor Laboratory Press, Cold Spring Harbor, N.Y.; Duda, Pattern Classification, Second Edition, 2001, John Wiley & Sons, Inc., pp. 259, 262-265; and Hastie, 2001, The Elements of Statistical Learning, Springer, New York; and Furey et al., 2000, Bioinformatics 16, 906-914. When used for classification, SVMs separate a given set of binary labeled data with a hyper-plane that is maximally distant from the labeled data. For cases in which no linear separation is possible, SVMs can work in combination with the technique of 'kernels', which automatically realizes a non-linear mapping to a feature space. The hyper-plane found by the SVM in feature space corresponds to a non-linear decision boundary in the input space.

Decision trees are described generally by Duda, 2001, Pattern Classification, John Wiley & Sons, Inc., New York, pp. 395-396. Tree-based methods partition the feature space into a set of rectangles, and then fit a model (like a constant) in each one. In some examples, the decision tree is random forest regression. One specific algorithm that can be used is a classification and regression tree (CART). Other specific decision tree algorithms include, but are not limited to, ID3, C4.5, MART, and Random Forests. CART, ID3, and C4.5 are described in Duda, 2001, Pattern Classification, John Wiley & Sons, Inc., New York. pp. 396-408 and pp. 411-412. CART, MART, and C4.5 are described in Hastie et al., 2001, The Elements of Statistical Learning, Springer-Verlag, New York, Chapter 9. Random Forests are described in Breiman, 1999, "Random Forests-Random Features," Technical Report 567, Statistics Department, U.C. Berkeley, September 1999.

Clustering (e.g., unsupervised clustering model algorithms and supervised clustering model algorithms) is described at pages 211-256 of Duda and Hart, Pattern Classification and Scene Analysis, 1973, John Wiley & Sons, Inc., New York, (hereinafter "Duda 1973"). As described in Section 6.7 of Duda 1973, the clustering problem is described as one of finding natural groupings in a dataset. To identify natural groupings, two issues are addressed. First, a way to measure similarity (or dissimilarity) between two samples is determined. This metric (similarity measure) is used to ensure that the samples in one cluster are more like one another than they are to samples in other clusters. Second, a mechanism for partitioning the data into clusters using the similarity measure is determined. Similarity measures are discussed in Section 6.7 of Duda 1973, where it is stated that one way to begin a clustering investigation is to define a distance function and to compute the matrix of distances between all pairs of samples in the training set. If distance is a good measure of similarity, then the distance between reference entities in the same cluster will be significantly less than the distance between the reference entities in different clusters. However, as stated on page 215 of Duda 1973, clustering does not require the use of a distance metric. For example, a nonmetric similarity function $s(x, x')$ can be used to compare two vectors $x$ and $x'$. Conventionally, $s(x, x')$ is a symmetric function whose value is large when $x$ and $x'$ are somehow "similar." An example of a nonmetric similarity function $s(x, x')$ is provided on page 218 of Duda 1973. Once a method for measuring "similarity" or "dissimilarity" between points in a dataset has been selected, clustering requires a criterion function that measures the clustering quality of any partition of the data. Partitions of the data set that extremize the criterion function are used to cluster the data. *See* page 217 of Duda 1973. Criterion functions are discussed in Section 6.8 of Duda 1973. More recently, Duda et al., Pattern Classification, 2nd edition, John Wiley & Sons, Inc. New York, has been published. Pages 537-563 describe clustering in detail. More information on clustering techniques can be found in Kaufman and Rousseeuw, 1990, Finding Groups in Data: An Introduction to Cluster Analysis, Wiley, New York, N.Y.; Everitt, 1993, Cluster analysis (3d ed.), Wiley, New York, N.Y.; and Backer, 1995, Computer-Assisted Reasoning in Cluster Analysis, Prentice Hall, Upper Saddle River, New Jersey. Particular exemplary clustering techniques that can be used in the present disclosure include, but are not limited to, hierarchical clustering (agglomerative clustering using nearest-neighbor algorithm, farthest-neighbor algorithm, the average linkage algorithm, the centroid algorithm, or the sum-of-squares algorithm), k-means clustering, fuzzy k-means clustering algorithm, and Jarvis-Patrick clustering. In some examples, the clustering comprises unsupervised clustering, where no preconceived notion of what clusters should form when the training set is clustered, are imposed.

Regression models, such as that of the multicategory logit models, are described in Agresti, An Introduction to Categorical Data Analysis, 1996, John Wiley & Sons, Inc., New York, Chapter 8. In some examples, the model makes use of a regression model disclosed in Hastie et al., 2001, The Elements of Statistical Learning, Springer-Verlag, New York. In some examples, gradient-boosting models are used toward, for example, the classification algorithms described herein; these gradient-boosting models are described in Boehmke, Bradley; Greenwell, Brandon (2019). "Gradient Boosting". Hands-On Machine Learning with R. Chapman & Hall. pp. 221-245. ISBN 978-1-138-49568-5. In some examples, ensemble modeling techniques are used, for example, toward the classification algorithms described herein; these ensemble modeling techniques are described in the implementation of classification models herein, are described in Zhou Zhihua (2012). Ensemble Methods: Foundations and Algorithms. Chapman and Hall/CRC. ISBN 978-1-439-83003-1.

In some examples, the machine learning analysis is performed by a device executing one or more programs (e.g., one or more programs stored in the Non-Persistent Memory 111 or in the Persistent Memory 112 in Figure 1) including instructions to perform the data analysis. In some examples, the data analysis is performed by a system comprising at least one processor *(e.g.,* the processing core 102) and memory *(e.g.,* one or more programs stored in the Non-Persistent Memory 111 or in the Persistent Memory 112) comprising instructions to perform the data analysis.

## Computer systems

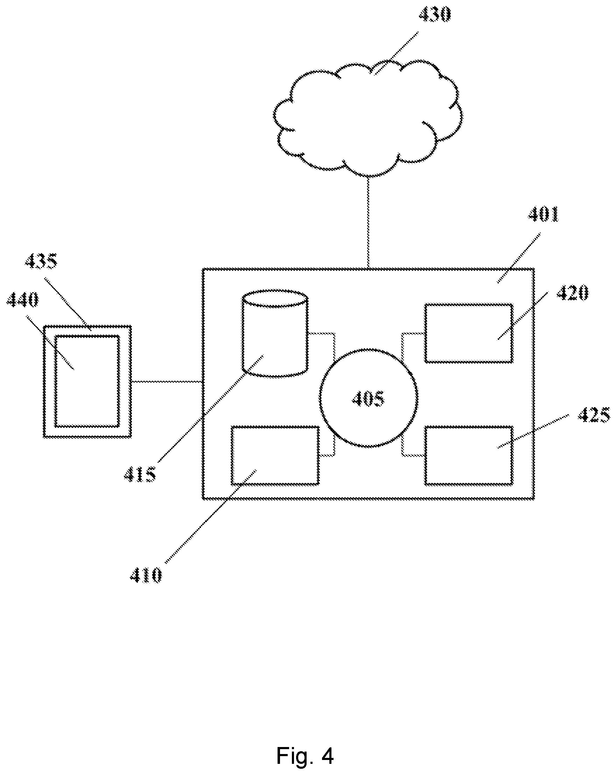

The present disclosure provides computer systems that are programmed to implement methods of the disclosure. FIG. 4 shows a computer system 401 that is programmed or otherwise configured to, for example, obtain a Raman signature of tooth samples, analyze the Raman spectra spatially across tooth samples, generate a temporal Raman profile, process data using trained models, and predict a subject's diagnostic status with respect to a disease or disorder. The computer system 401 can regulate various aspects of sensor data analysis of the present disclosure, such as, for example, staining a tooth sample, obtaining a fluorescence image of stained tooth samples, analyzing a fluorescence intensity spatially across stained tooth samples, generating a temporal Raman profile, measuring the dynamics of the temporal profile, process data using trained models, and predicting a subject's diagnostic status with respect to a disease or disorder. The computer system 401 can be an electronic device of a user or a computer system that is remotely located with respect to the electronic device. The electronic device can be a mobile electronic device.

The computer system 401 includes a central processing unit (CPU, also "processor" and "computer processor" herein) 405, which can be a single core or multi core processor, or a plurality of processors for parallel processing. The computer system 401 also includes memory or memory location 410 (e.g., random-access memory, read-only memory, flash memory), electronic storage unit 415 (e.g., hard disk), communication interface 420 (e.g., network adapter) for communicating with one or more other systems, and peripheral devices 425, such as cache, other memory, data storage and/or electronic display adapters. The memory 410, storage unit 415, interface 420 and peripheral devices 425 are in communication with the CPU 405 through a communication bus (solid lines), such as a motherboard. The storage unit 415 can be a data storage unit (or data repository) for storing data. The computer system 401 can be operatively coupled to a computer network ("network") 430 with the aid of the communication interface 420. The network 430 can be the Internet, an internet and/or extranet, or an intranet and/or extranet that is in communication with the Internet. The network 430 in some cases is a telecommunication and/or data network. The network 430 can include one or more computer servers, which can enable distributed computing, such as cloud computing. The network 430, in some cases with the aid of the computer system 401, can implement a peer-to-peer network, which may enable devices coupled to the computer system 401 to behave as a client or a server.

The CPU 405 can execute a sequence of machine-readable instructions, which can be embodied in a program or software. The instructions may be stored in a memory location, such as the memory 410. The instructions can be directed to the CPU 405, which can subsequently program or otherwise configure the CPU 405 to implement methods of the present disclosure. Examples of operations performed by the CPU 405 can include fetch, decode, execute, and writeback.

The CPU 405 can be part of a circuit, such as an integrated circuit. One or more other components of the system 401 can be included in the circuit. In some cases, the circuit is an application specific integrated circuit (ASIC).

The storage unit 415 can store files, such as drivers, libraries and saved programs. The storage unit 415 can store user data, e.g., user preferences and user programs. The computer system 401 in some cases can include one or more additional data storage units that are external to the computer system 401, such as located on a remote server that is in communication with the computer system 401 through an intranet or the Internet.

The computer system 401 can communicate with one or more remote computer systems through the network 430. For instance, the computer system 401 can communicate with a remote computer system of a user (e.g., a health care provider). Examples of remote computer systems include personal computers (e.g., portable PC), slate or tablet PC's (e.g., Apple® iPad, Samsung® Galaxy Tab), telephones, Smart phones (e.g., Apple® iPhone, Android-enabled device, Blackberry®), or personal digital assistants. The user can access the computer system 401 via the network 430.

Methods as described herein can be implemented by way of machine (e.g., computer processor) executable code stored on an electronic storage location of the computer system 401, such as, for example, on the memory 410 or electronic storage unit 415. The machine executable or machine-readable code can be provided in the form of software. During use, the code can be executed by the processor 405. In some cases, the code can be retrieved from the storage unit 415 and stored on the memory 410 for ready access by the processor 405. In some situations, the electronic storage unit 415 can be precluded, and machine-executable instructions are stored on memory 410.

The code can be pre-compiled and configured for use with a machine having a processer adapted to execute the code, or can be compiled during runtime. The code can be supplied in a programming language that can be selected to enable the code to execute in a precompiled or as-compiled fashion.

Aspects of the systems and methods provided herein, such as the computer system 401, can be embodied in programming. Various aspects of the technology may be thought of as "products" or "articles of manufacture" typically in the form of machine (or processor) executable code and/or associated data that is carried on or embodied in a type of machine readable medium. Machine-executable code can be stored on an electronic storage unit, such as memory (e.g., read-only memory, random-access memory, flash memory) or a hard disk. "Storage" type media can include any or all of the tangible memory of the computers, processors or the like, or associated modules thereof, such as various semiconductor memories, tape drives, disk drives and the like, which may provide non-transitory storage at any time for the software programming. All or portions of the software may at times be communicated through the Internet or various other telecommunication networks. Such communications, for example, may enable loading of the software from one computer or processor into another, for example, from a management server or host computer into the computer platform of an application server. Thus, another type of media that may bear the software elements includes optical, electrical and electromagnetic waves, such as used across physical interfaces between local devices, through wired and optical landline networks and over various air-links. The physical elements that carry such waves, such as wired or wireless links, optical links or the like, also may be considered as media bearing the software. As used herein, unless restricted to non-transitory, tangible "storage" media, terms such as computer or machine "readable medium" refer to any medium that participates in providing instructions to a processor for execution.

Hence, a machine readable medium, such as computer-executable code, may take many forms, including but not limited to, a tangible storage medium, a carrier wave medium or physical transmission medium. Non-volatile storage media include, for example, optical or magnetic disks, such as any of the storage devices in any computer(s) or the like, such as may be used to implement the databases, etc. shown in the drawings. Volatile storage media include dynamic memory, such as main memory of such a computer platform. Tangible transmission media include coaxial cables; copper wire and fiber optics, including the wires that comprise a bus within a computer system. Carrier-wave transmission media may take the form of electric or electromagnetic signals, or acoustic or light waves such as those generated during radio frequency (RF) and infrared (IR) data communications. Common forms of computer-readable media therefore include for example: a floppy disk, a flexible disk, hard disk, magnetic tape, any other magnetic medium, a CD-ROM, DVD or DVD-ROM, any other optical medium, punch cards paper tape, any other physical storage medium with patterns of holes, a RAM, a ROM, a PROM and EPROM, a FLASH-EPROM, any other memory chip or cartridge, a carrier wave transporting data or instructions, cables or links transporting such a carrier wave, or any other medium from which a computer may read programming code and/or data. Many of these forms of computer readable media may be involved in carrying one or more sequences of one or more instructions to a processor for execution.

The computer system 401 can include or be in communication with an electronic display 435 that comprises a user interface (UI) 440 for providing, for example, Raman image data, Raman spectral data, temporal Raman profiles, and models. Examples of UI's include, without limitation, a graphical user interface (GUI) and web-based user interface.

Methods and systems of the present disclosure can be implemented by way of one or more algorithms. An algorithm can be implemented by way of software upon execution by the central processing unit 405. The algorithm can, for example, obtain a Raman image of tooth samples, analyze Raman spectra spatially across tooth samples, generate a temporal Raman profile, process data using trained models, and predict a subject's diagnostic status with respect to a disease or disorder.

## EXAMPLES

### Example 1: Dynamic Raman spectroscopy profiles in tooth samples for determining Autism Spectrum Disorder (ASD) disease risk

Using methods and systems of the present disclosure, dynamic Raman spectroscopy profiles in tooth samples were generated and subsequently analyzed to determine a disease risk in a subject. Generally, the temporal dynamics of biological response (e.g., physiological responses) were found to be imprinted in samples (e.g., tooth samples), and can be analyzed to determine disease risk in a subject. Dynamic Raman spectroscopy profiles were generated during a time period that comprised fetal (prenatal) development and early childhood in two sets of children-a first set with autism spectrum disorder and a second set without autism spectrum disorder (ASD). The dynamic Raman spectroscopy profiles were analyzed to reveal novel features therein, which accurately distinguished the autism cases from controls. For example, early life spectroscopic signatures were found to reveal a disease risk of ASD in later life. As a comparison, a clinical diagnosis of autism is usually determined around the age of 3 to 4 years.

A primary tooth sample was obtained from each child subject. The tooth samples were sectioned open and Raman spectroscopy signals were measured on the tooth samples in order to develop temporal Raman spectroscopy profiles indicative of physiological response over the prenatal and postnatal period. The temporal profiles were analyzed using machine learning algorithms of the present disclosure to train highly accurate classifiers to determine disease risk (e.g., autism).

FIG. 5 shows an example of classifier accuracy of diagnosing autism spectrum disorder (ASD) utilizing features derived from application of RQA to ICA-derived dimensions of the Raman waveform, as indicated by an experimental Receiver Operating Characteristics (ROC) curve for evaluating accuracy of the disclosed method of evaluating a subject for autism spectrum disorder. A ROC curve can be used for evaluating a performance of a binary classifier. A ROC curve is plotted as sensitivity (also called as a true positive rate) against specificity (also called as a true negative rate). A perfect classifier may have a 100% sensitivity and 100% specificity and an Area-Under-the-Curve (AUC) of 1.0. As shown in FIG. 5, the classifier configured to determine the presence of ASD in a subject based on dynamic Raman RQA dynamic profile had an Area-Under-the-Curve (AUC) of the receiver operating characteristic (ROC) of 0.861, with a 95% confidence interval (CI) of 0.769 to 0.954. The receiver operating characteristic (ROC) shows how sensitivity and specificity values of the classifier change as varying thresholds are assigned to probabilistic projections.

Therefore, analysis of Raman spectroscopic signatures using methods and systems of the present disclosure successfully determined the disease risk of autism with greater than 0.86 AUC, using only spectroscopic signatures measured on non-invasively obtained biological samples (e.g., tooth samples) from child subjects. These results demonstrate that dynamics of human physiology in early life are linked to disease later on, which can be accurately detected and profiled using methods and systems of the present disclosure.

**Example 2: Dynamic Raman spectroscopy profiles in tooth samples for determining Amyotrophic Lateral Sclerosis disease risk**

Using methods and systems of the present disclosure, dynamic Raman spectroscopy profiles in tooth samples were generated and subsequently analyzed to determine a disease risk in a subject. Generally, the temporal dynamics of biological response (e.g., physiological responses) were found to be imprinted in samples (e.g., tooth samples), and can be analyzed to determine disease risk in a subject. Dynamic Raman spectroscopy profiles were generated during a time period that comprised early childhood and adolescence in two sets of adults-a first set with amyotrophic lateral sclerosis (ALS) and a second set without ALS. The dynamic Raman spectroscopy profiles were analyzed to reveal novel features therein, which accurately distinguished the ALS cases from controls. For example, early life spectroscopic signatures were found to reveal a disease risk of ALS in later life.

A permanent tooth sample was obtained from each adult subject. The tooth samples were sectioned open and Raman spectroscopy signals were measured on the tooth samples in order to develop temporal Raman spectroscopy profiles indicative of physiological response over the early childhood and adolescence period. The temporal profiles were analyzed using machine learning algorithms of the present disclosure to train highly accurate classifiers to determine disease risk (e.g., ALS).

**FIG.** 6 shows an example of classifier accuracy of diagnosing ALS utilizing features derived from application of RQA to ICA-derived dimensions of the Raman waveform, as indicated by an experimental Receiver Operating Characteristics (ROC) curve for evaluating accuracy of the disclosed method of evaluating a subject for autism spectrum disorder. A ROC curve can be used for evaluating a performance of a binary classifier. A ROC curve is plotted as sensitivity (also called as a true positive rate) against specificity (also called as a true negative rate). A perfect classifier may have a 100% sensitivity and 100% specificity and an Area-Under-the-Curve (AUC) of 1.0. As shown in FIG. 6, the classifier configured to determine the presence of ASD in a subject based on dynamic Raman RQA dynamic profile had an Area-Under-the-Curve (AUC) of the receiver operating characteristic (ROC) of 0.880, with a 95% confidence interval (CI) of 0.658 to 1.000. The receiver operating characteristic (ROC) shows how sensitivity and specificity values of the classifier change as varying thresholds are assigned to probabilistic projections.

Therefore, analysis of Raman spectroscopic signatures using methods and systems of the present disclosure successfully determined the disease risk of ALS with greater than 0.88 AUC, using only spectroscopic signatures measured on biological samples (e.g., tooth samples) from adults. These results demonstrate that dynamics of human physiology in early life are linked to disease later on, which can be accurately detected and profiled using methods and systems of the present disclosure.

**Claims**

1. A device comprising one or more processors, and memory storing one or more programs for execution by the one or more processors, the one or more programs comprising instructions for:

   (a) sampling each respective position in a plurality of positions along a reference line on a tooth sample obtained from a subject associated with a Raman signature of the subject, thereby obtaining a plurality of Raman spectra, each Raman spectrum in the plurality of Raman spectra corresponding to a different position in the plurality of positions, and each position in the plurality of positions representing a different period of growth of the tooth sample associated with the Raman signature;
   (b) analyzing each of the plurality of Raman spectra across the reference line on the tooth sample thereby obtaining a first dataset;
   (c) deriving a respective second dataset of a set of features from the plurality of Raman spectra, each respective feature in the set of features being determined by a sequential variation in the Raman spectra of the plurality of Raman spectra measurements; and

(d) processing the set of features using a trained model to predict a subject's diagnostic status with respect to disease or disorder associated with the Raman signature, wherein the disease or disorder comprises autism spectrum disorder (ASD) or amyotrophic lateral sclerosis (ALS).

2. The device of claim 1, wherein the instructions further perform detecting or monitoring changes in the plurality of Raman spectra across the plurality of positions indicative of a temporal response of the subject.

3. The device of claim 2, wherein the temporal response comprises a biological response, a physiological response, an anatomical response, a treatment response, a stress-related response, or a combination thereof.

4. The device of any one of claims 1-3, wherein the plurality of Raman spectra comprises from 200 to 3700 wave numbers.

5. The device of any one of claims 1-4, wherein sampling comprises using a Raman spectroscopy microscope, optionally wherein the Raman spectroscopy microscope comprises a 50X air coupled objective, a 63X water immersion coupled objective, or any combination thereof

6. The device of any one of claims 1-5, wherein the sampling comprises exposing the tooth sample to a light source to generate the Raman spectra of the plurality of Raman spectra at the plurality of positions, optionally wherein the light source comprises a laser, and optionally wherein the laser comprises a wavelength of 785 nm, a wavelength of 532 nm, or any combination thereof.

7. The device of any one of claims 1-6, wherein the instructions further perform translating the tooth sample, wherein the translating comprises moving the tooth sample with a step size of 2 microns to 5 microns from a first position to a second position of the plurality of positions subsequent to acquiring a Raman spectrum of the plurality of Raman spectra, optionally wherein the translating is performed using an integration time of 0.2 seconds to 0.3 seconds.

8. The device of any one of claims 1-7, wherein the disease or disorder comprises autism spectrum disorder (ASD).

9. The device of any one of claims 1-7, wherein the disease or disorder comprises amyotrophic lateral sclerosis (ALS).

10. The device of any one of claims 1-9, wherein predicting the subject's diagnostic status with respect to the disease or disorder comprises processing changes in the Raman spectra across the plurality of positions with the trained model.

11. The device of claim 10, wherein the trained model is selected from the group consisting of: a neural network algorithm, a support vector machine algorithm, a decision tree algorithm, an unsupervised clustering algorithm, a supervised clustering algorithm, a regression algorithm, a gradient-boosting algorithm, a gradient-boosted ensemble model, and any combination thereof.

12. The device of claim 10, wherein the trained model is configured to process one or more features selected from the group consisting of laminarity, entropy, trapping time (TT), mean diagonal length (MDL), recurrence time (RT), Vmax, determinism, Lmax, and any combination thereof.

13. The device of any one of claims 1-12, wherein the trained model predicts diagnostic status with respect to the disease or disorder with a sensitivity of at least 80%, a specificity of at least 80%, a positive predictive value of at least 80%, a negative predictive value of at least 80%, an Area Under the Receiver Operating Characteristic (AUROC) of at least 0.80, or any combination thereof.

14. The device of any one of claims 1-13, wherein the set of features of the plurality of Raman spectra comprises variation of an abundance of one or more substances in the Raman spectra of the plurality of Raman spectra across the different positions in the plurality of positions.

15. A non-transitory computer readable storage medium and one or more computer programs embedded therein for classification, the one or more computer programs comprising instructions which, when executed by a computer system, cause the computer system to perform a method comprising:

(a) sampling each respective position in a plurality of positions along a reference line on a tooth sample obtained from a subject associated with a Raman signature of the subject, thereby obtaining a plurality of Raman spectra, each Raman spectra in the plurality of Raman spectra corresponding to a different position in the plurality of positions, and each position in the plurality of positions representing a different period of growth of the tooth sample associated with the Raman signature;

(b) analyzing each of the plurality of Raman spectra across the reference line on the tooth sample thereby obtaining a first dataset;

(c) deriving a respective second dataset of a set of features from the plurality of Raman spectra, each respective feature in the set of features being determined by a sequential variation in the Raman spectra of the plurality of Raman spectra measurements; and

(d) processing the set of features using a trained model to predict a subject's diagnostic status with respect to disease or disorder associated with the Raman signature, wherein the disease or disorder comprises autism spectrum disorder (ASD) or amyotrophic lateral sclerosis (ALS).

**Patentansprüche**

1. Eine Vorrichtung umfassend einen oder mehr Prozessoren und einen Speicher, der ein oder mehr Programme zur Ausführung durch die ein oder mehr Prozessoren speichert, wobei die ein oder mehr Programme Anweisungen umfassen zum:

(a) Untersuchen jeder jeweiligen Position in einer Vielzahl von Positionen entlang einer Referenzlinie auf einer von einem Subjekt erhaltenen Zahnprobe, die mit einer Raman-Signatur des Subjekts assoziiert ist, dadurch Erhalten einer Vielzahl von Raman-Spektra, wobei jedes Raman-Spektrum in der Vielzahl von Raman-Spektra einer unterschiedlichen Position in der Vielzahl von Positionen entspricht, und jede Position in der Vielzahl von Positionen eine unterschiedliche Wachstumsperiode der mit der Raman-Signatur assoziierten Zahnprobe darstellt;

(b) Analysieren jedes der Vielzahl von Raman-Spektra entlang der Referenzlinie auf der Zahnprobe, dadurch Erhalten eines ersten Datensatzes;

(c) Ableiten eines jeweiligen zweiten Datensatzes eines Satzes von Merkmalen aus der Vielzahl von Raman-Spektra, wobei jedes jeweilige Merkmal im Satz von Merkmalen durch eine fortlaufende Variation in den Raman-Spektra aus der Vielzahl von Raman-Spektrummessungen bestimmt wird; und

(d) Verarbeiten des Satzes von Merkmalen mittels eines trainierten Modells, um den diagnostischen Status eines Subjekts bezüglich der mit der Raman-Signatur assoziierten Krankheit oder Störung vorherzusagen, wobei die Krankheit oder Störung Autismus-Spektrum-Störung (ASD) oder amyotrophe Lateralsklerose (ALS) umfasst.

2. Die Vorrichtung aus Anspruch 1, wobei die Anweisungen weiter Nachweisen oder Beobachten von Änderungen in der Vielzahl von Raman-Spektra über die Vielzahl der Positionen durchführen, die eine zeitliche Antwort des Subjekts anzeigen.

3. Die Vorrichtung aus Anspruch 1, wobei die zeitliche Antwort eine biologische Antwort, eine physiologische Antwort, eine anatomische Antwort, eine Behandlungsantwort, eine stressbezogene Antwort oder eine Kombination davon umfasst.

4. Die Vorrichtung aus irgendeinem der Ansprüche 1-3, wobei die Vielzahl von Raman-Spektra von 200 bis 3700 Wellenzahlen umfasst.

5. Die Vorrichtung aus irgendeinem der Ansprüche 1-4, wobei das Untersuchen die Verwendung eines Raman-Spektroskopie-Mikroskops umfasst, optional wobei das Raman-Spektroskopie-Mikroskop ein 50X luftgekoppeltes Objektiv, ein 63X Wasserimmersiongekoppeltes Objektiv oder jegliche Kombination davon umfasst.

6. Die Vorrichtung aus irgendeinem der Ansprüche 1-5, wobei das Untersuchen das Aussetzen der Zahnprobe gegenüber einer Lichtquelle umfasst, um die Raman-Spektra in der Vielzahl von Raman-Spektra an der Vielzahl von Positionen zu erzeugen, optional wobei die Lichtquelle einen Laser umfasst, und optional wobei der Laser eine Wellenlänge von 785 nm, eine Wellenlänge von 532 nm oder jegliche Kombination davon umfasst.

7. Die Vorrichtung aus irgendeinem der Ansprüche 1-6, wobei die Anweisungen weiter das Verschieben der Zahnprobe umfassen, wobei das Verschieben das Bewegen der Zahnprobe mit einer Schrittlänge von 2 Mikrometer bis 5 Mikrometer aus einer ersten Position in eine zweite Position aus der Vielzahl von Positionen nach Aufnahme eines Raman-Spektrums in der Vielzahl von Raman-Spektra umfasst, optional wobei das Verschieben unter Verwendung einer Integrationszeit von 0,2 Sekunden bis 0,3 Sekunden durchgeführt wird.

8. Die Vorrichtung aus irgendeinem der Ansprüche 1-7, wobei die Krankheit oder Störung Autismus-Spektrum-Störung (ASD) umfasst.

9. Die Vorrichtung aus irgendeinem der Ansprüche 1-7, wobei die Krankheit oder Störung amyotrophe Lateralsklerose (ALS) umfasst.

10. Die Vorrichtung aus irgendeinem der Ansprüche 1-9, wobei das Vorhersagen des diagnostischen Status der Subjekts bezüglich der Krankheit oder Störung Verarbeiten von Veränderungen in den Raman-Spektra über die Vielzahl von Positionen mit dem trainierten Modell umfasst.

11. Die Vorrichtung aus Anspruch 10, wobei das trainierte Modell ausgewählt ist aus der Gruppe bestehend aus: einem neuralen Netzwerkalgorithmus, einem Support-Vektor-Maschinen-Algorithmus, einem nicht überwachten Clustering-Algorithmus, einem überwachten Clustering-Algorithmus, einem Regressionsalgorithmus, einem Gradient-Boosting-Algorithmus, einem Gradient-Boosting-Ensemble-Modell und jeglicher Kombination davon.

12. Die Vorrichtung aus Anspruch 10, wobei das trainierte Modell konfiguriert ist, ein oder mehr Merkmale ausgewählt aus der Gruppe bestehend aus Laminarität, Entropie, Einfangzeit (TT), mittlere Diagonallänge (MDL), Wiederkehrzeit (RT), Vmax, Determinismus, Lmax und jeglicher Kombination davon zu verarbeiten.

13. Die Vorrichtung aus irgendeinem der Ansprüche 1-12, wobei das trainierte Modell den diagnostischen Status mit einer Sensitivität von mindestens 80%, einer Spezifität von mindestens 80%, einem positiven Vorhersagewert von mindestens 80%, einem negativen Vorhersagewert von mindestens 80%, einer Kurve unter der Empfänger-Operationscharakteristik (AUROC) von mindestens 0,80 oder jeglicher Kombination davon vorhersagt.

14. Die Vorrichtung aus irgendeinem der Ansprüche 1-13, wobei der Satz von Merkmalen in der Vielzahl von Raman-Spektra eine Variation der Häufigkeit von einer oder mehr Substanzen in den Raman-Spektra in der Vielzahl von Raman-Spektra über die verschiedenen Positionen in der Vielzahl von Positionen umfasst.

15. Ein nichtvergängliches computerlesbares Speichermedium und ein oder mehr darin eingebettete Computerprogramme zur Klassifizierung, wobei die ein oder mehr Computerprogramme Anweisungen umfassen, die, wenn sie von einem Computersystem ausgeführt werden, das Computersystem veranlassen, ein Verfahren durchzuführen umfassend:

(a) Untersuchen jeder jeweiligen Position in einer Vielzahl von Positionen entlang einer Referenzlinie auf einer von einem Subjekt erhaltenen Zahnprobe, die mit einer Raman-Signatur des Subjekts assoziiert ist, dadurch Erhalten einer Vielzahl von Raman-Spektra, wobei jedes Raman-Spektrum in der Vielzahl von Raman-Spektra einer unterschiedlichen Position in der Vielzahl von Positionen entspricht, und jede Position in der Vielzahl von Positionen eine unterschiedliche Wachstumsperiode der mit der Raman-Signatur assoziierten Zahnprobe darstellt;
(b) Analysieren jedes der Vielzahl von Raman-Spektra entlang der Referenzlinie auf der Zahnprobe, dadurch Erhalten eines ersten Datensatzes;
(c) Ableiten eines jeweiligen zweiten Datensatzes eines Satzes von Merkmalen aus der Vielzahl von Raman-Spektra, wobei jedes jeweilige Merkmal im Satz von Merkmalen durch eine fortlaufende Variation in den Raman-Spektra aus der Vielzahl von Raman-Spektrummessungen bestimmt wird; und
(d) Verarbeiten des Satzes von Merkmalen mittels eines trainierten Modells, um den diagnostischen Status eines Subjekts bezüglich der mit der Raman-Signatur assoziierten Krankheit oder Störung vorherzusagen, wobei die Krankheit oder Störung Autismus-Spektrum-Störung (ASD) oder amyotrophe Lateralsklerose (ALS) umfasst.

## Revendications

1. Dispositif comprenant un ou plusieurs processeurs, et une mémoire stockant un ou plusieurs programmes pour l'exécution par les un ou plusieurs processeurs, les un ou plusieurs programmes comprenant des instructions pour :

(a) l'échantillonnage de chaque position respective dans une pluralité de positions le long d'une ligne de référence sur un échantillon dentaire obtenu à partir d'un sujet associé à une signature Raman du sujet, ainsi obtenant une pluralité de spectres Raman, chaque spectre Raman dans la pluralité de spectres Raman correspondant à une position différente dans la pluralité de positions, et chaque position dans la pluralité de positions représentant une période de croissance différente de l'échantillon dentaire associé à la signature Raman ;
(b) l'analyse de chacun de la pluralité de spectres Raman sur la ligne de référence sur l'échantillon dentaire, ainsi obtenant un premier ensemble de données ;
(c) la dérivation d'un second ensemble de données respectif d'un ensemble de caractéristiques à partir de la pluralité de spectres Raman, chaque caractéristique respective dans l'ensemble de caractéristiques étant déterminée par une variation séquentielle dans les spectres Raman de la pluralité de mesures de spectres Raman ; et
(d) le traitement de l'ensemble de caractéristiques à l'aide d'un modèle entraîné pour prédire l'état diagnostique d'un sujet par rapport à une maladie ou à un trouble associé(e) à la signature Raman, dans lequel la maladie ou le trouble comprend un trouble du spectre autistique (ASD) ou une sclérose latérale amyotrophique (ALS).

2. Dispositif selon la revendication 1, dans lequel les instructions réalisent en outre la détection ou la surveillance de changements dans la pluralité de spectres Raman à travers la pluralité de positions indiquant une réponse temporelle du sujet.

3. Dispositif selon la revendication 2, dans lequel la réponse temporelle comprend une réponse biologique, une réponse physiologique, une réponse anatomique, une réponse de traitement, une réponse liée au stress, ou une combinaison de celles-ci.

4. Dispositif selon l'une quelconque des revendications 1 à 3, dans lequel la pluralité de spectres Raman comprend de 200 à 3 700 nombres d'ondes.

5. Dispositif selon l'une quelconque des revendications 1 à 4, dans lequel l'échantillonnage comprend l'utilisation d'un microscope de spectroscopie Raman, facultativement dans lequel le microscope de spectroscopie Raman comprend un objectif 50X couplé à l'air, un objectif 63X couplé par immersion dans l'eau, ou toute combinaison de ceux-ci

6. Dispositif selon l'une quelconque des revendications 1 à 5, dans lequel l'échantillonnage comprend l'exposition de l'échantillon dentaire à une source de lumière pour générer les spectres Raman de la pluralité de spectres Raman à la pluralité de positions, facultativement dans lequel la source de lumière comprend un laser, et facultativement dans lequel le laser comprend une longueur d'onde de 785 nm, une longueur d'onde de 532 nm, ou toute combinaison de celles-ci.

7. Dispositif selon l'une quelconque des revendications 1 à 6, dans lequel les instructions réalisent en outre la translation de l'échantillon dentaire, dans lequel la translation comprend le déplacement de l'échantillon dentaire avec une taille de pas de 2 microns à 5 microns d'une première position à une seconde position de la pluralité de positions suite à l'acquisition d'un spectre Raman de la pluralité de spectres Raman, facultativement dans lequel la translation est réalisée en utilisant un temps d'intégration de 0,2 seconde à 0,3 seconde.

8. Dispositif selon l'une quelconque des revendications 1 à 7, dans lequel la maladie ou le trouble comprend un trouble du spectre autistique (ASD).

9. Dispositif selon l'une quelconque des revendications 1 à 7, dans lequel la maladie ou le trouble comprend une sclérose latérale amyotrophique (ALS).

10. Dispositif selon l'une quelconque des revendications 1-9, dans lequel la prédiction de l'état diagnostique du sujet par rapport à la maladie ou au trouble comprend le traitement de changements dans les spectres Raman à travers la pluralité de positions à l'aide du modèle entraîné.

11. Dispositif selon la revendication 10, dans lequel le modèle entraîné est sélectionné dans le groupe consistant en : un algorithme de réseau neuronal, un algorithme de machine à vecteurs de support, un algorithme d'arbre de décision, un algorithme de groupement non supervisé, un algorithme de groupement supervisé, un algorithme de régression, un algorithme d'amplification de gradient, un modèle à amplification de gradient, et toute combinaison de ceux-ci.

12. Dispositif selon la revendication 10, dans lequel le modèle entraîné est configuré pour traiter une ou plusieurs caractéristiques sélectionnées dans le groupe consistant en la laminarité, l'entropie, le temps de piégeage (TT), la longueur diagonale moyenne (MDL), le temps de récurrence (RT), Vmax, le déterminisme, Lmax, et toute combinaison de ceux-ci.

13. Dispositif selon l'une quelconque des revendications 1 à 12, dans lequel le modèle entraîné prédit l'état diagnostique par rapport à la maladie ou au trouble avec une sensibilité d'au moins 80 %, une spécificité d'au moins 80 %, une valeur prédictive positive d'au moins 80 %, une valeur prédictive négative d'au moins 80 %, une surface sous la caractéristique de fonctionnement du récepteur (AUROC) d'au moins 0,80, ou toute combinaison de celles-ci.

14. Dispositif selon l'une quelconque des revendications 1 à 13, dans lequel l'ensemble de caractéristiques de la pluralité de spectres Raman comprend une variation d'une abondance d'une ou plusieurs substances dans les spectres Raman de la pluralité de spectres Raman à travers les différentes positions dans la pluralité de positions.

15. Support de stockage non transitoire lisible par ordinateur et un ou plusieurs programmes informatiques incorporés dans celui-ci à des fins de classification, les un ou plusieurs programmes informatiques comprenant des instructions qui, lorsqu'elles sont exécutées par un système informatique, amènent le système informatique à exécuter un procédé comprenant :

(a) l'échantillonnage de chaque position respective dans une pluralité de positions le long d'une ligne de référence sur un échantillon dentaire obtenu à partir d'un sujet associé à une signature Raman du sujet, ainsi obtenant une pluralité de spectres Raman, chaque spectre Raman dans la pluralité de spectres Raman correspondant à une position différente dans la pluralité de positions, et chaque position dans la pluralité de positions représentant une période de croissance différente de l'échantillon dentaire associé à la signature Raman ;

(b) l'analyse de chacun de la pluralité de spectres Raman sur la ligne de référence sur l'échantillon dentaire, ainsi obtenant un premier ensemble de données ;

(c) la dérivation d'un second ensemble de données respectif d'un ensemble de caractéristiques à partir de la pluralité de spectres Raman, chaque caractéristique respective dans l'ensemble de caractéristiques étant déterminée par une variation séquentielle dans les spectres Raman de la pluralité de mesures de spectres Raman ; et

(d) le traitement de l'ensemble de caractéristiques à l'aide d'un modèle entraîné pour prédire l'état diagnostique d'un sujet par rapport à une maladie ou à un trouble associé(e) à la signature Raman, dans lequel la maladie ou le trouble comprend un trouble du spectre autistique (ASD) ou une sclérose latérale amyotrophique (ALS).

System 100

Non-Persistent
Memory 111

| Operating system | 116 |
| Network communication module | 118 |
| Classifier training module | 120 |
| Dataset for biological samples from training subjects | 122 |

Processing core — 102

| | |
|---|---|
| Training subject 1 | 124-1 |

114

| Feature 1 | 126-1-1 |
| Feature 2 | 126-1-2 |
| • • • | |
| Feature M | 126-1-M |
| Diagnostic status | 128-1 |
| Training subject 2 | 124-2 |
| Feature 1 | 126-2-1 |
| Feature 2 | 126-2-2 |
| • • • | |
| Feature M | 126-2-M |
| Diagnostic status | 128-2 |

104

Network
interface

• • •

| Training subject Y | 124-Y |
| Classifier validation module | 130 |
| Dataset for biological samples from validation subjects | 132 |
| Patient classification module | 134 |

106

User interface

Display

108

Input

110

Persistent memory 112

Fig. 1

Fig. 2A

EP 4 256 310 B1

Fig. 2B

EP 4 256 310 B1

Fig. 2C

EP 4 256 310 B1

Expose a biological sample of the subject to an optical signal comprising Raman wavelengths — 302

Acquire a plurality of Raman spectra from the exposed biological sample — 304

Process the plurality of Raman spectra to generate a spatial map of the Raman spectra — 306

Determine the risk of the disease or disorder of the subject based at least in part on the spatial map of the Raman spectra — 308

300

Fig. 3

Fig. 4

Classification of ASD from Raman RQA Dynamics

AUC: 0.861 (0.769-0.954)

Specificity

Sensitivity

Fig. 5

Classification of ASD from Raman RQA Dynamics

AUC: 0.880 (0.658-1.000)

Fig. 6

## REFERENCES CITED IN THE DESCRIPTION

### Non-patent literature cited in the description

- **AUSTIN C. et al.** Uncovering system-specific stress signatures in primate teeth with multimodal imaging". *Nature Scientific Reports*, 2016, vol. 6 (1), 1-11
- **MORISHITA H. et al.** Tooth-Matrix Biomarkers to Reconstruct Critical Periods of Brain Plasticity. *TRENDS IN NEUROSCIENCE*, 2017, vol. 40 (1), 1-3
- **WEBBER et al.** Simpler Methods Do It Better: Success of Recurrence Quantification Analysis as a General Purpose Data Analysis Tool. *Physics Letters A*, 2009, vol. 373, 3753-3756
- **MARWAN et al.** Recurrence Plots for the Analysis of Complex Systems. *Physics Reports*, 2007, vol. 438, 237-239
- **VINCENT et al.** Stacked denoising autoencoders: Learning useful representations in a deep network with a local denoising criterion. *J Mach Learn Res*, 2010, vol. 11, 3371-3408
- **LAROCHELLE et al.** Exploring strategies for training deep neural networks. *J Mach Learn Res*, 2009, vol. 10, 1-40
- **HASSOUN**. *Fundamentals of Artificial Neural Networks, Massachusetts Institute of Technology*, 1995
- **LEE, T.-W.** Independent component analysis: Theory and applications. Mass: Kluwer Academic Publishers, 1998
- **HYVÄRINEN, A** ; **KARHUNEN, J.** ; **OJA, E.** Independent Component Analysis. Wiley, 2001
- Principal Component Analysis. **JOLLIFFE, I. T.** Springer Series in Statistics. Springer-Verlag, 2002
- **CRISTIANINI** ; **SHAWE-TAYLOR**. An Introduction to Support Vector Machines. Cambridge University Press, 2000
- A training algorithm for optimal margin classifiers. **BOSER et al.** Proceedings of the 5th Annual ACM Workshop on Computational Learning Theory. ACM Press, 1992, 142-152
- **VAPNIK**. Statistical Learning Theory. Wiley, 1998
- **MOUNT**. Bioinformatics: sequence and genome analysis. Cold Spring Harbor Laboratory Press, 2001
- **DUDA**. Pattern Classification. John Wiley & Sons, Inc, 2001, vol. 259, 262-265
- **HASTIE**. The Elements of Statistical Learning,. Springer, 2001
- **FUREY et al.** *Bioinformatics*, 2000, vol. 16, 906-914
- **DUDA**. Pattern Classification. John Wiley & Sons, Inc., 2001, 395-396
- **DUDA**. Pattern Classification. John Wiley & Sons, Inc, 2001, 396-408, 411-412
- **HASTIE et al.** The Elements of Statistical Learning. Springer-Verlag, 2001
- **BREIMAN**. Random Forests-Random Features. *Technical Report 567, Statistics Department*, September 1999
- **DUDA** ; **HART**. Pattern Classification and Scene Analysis. John Wiley & Sons, Inc., 1973, 211-256
- **DUDA et al.** Pattern Classification. John Wiley & Sons, Inc, 537-563
- **KAUFMAN** ; **ROUSSEEUW**. Finding Groups in Data: An Introduction to Cluster Analysis. Wiley, 1990
- **EVERITT**. Cluster analysis. Wiley, 1993
- **BACKER**. *Computer-Assisted Reasoning in Cluster Analysis, Prentice Hall, Upper Saddle River*, 1995
- **AGRESTI**. An Introduction to Categorical Data Analysis. John Wiley & Sons, Inc., 1996
- **BOEHMKE** ; **BRADLEY** ; **GREENWELL** ; **BRANDON**. Gradient Boosting. *Hands-On Machine Learning with R. Chapman & Hall*, 2019, ISSN 978-1-138-49568-5, 221-245
- **ZHOU ZHIHUA**. Ensemble Methods: Foundations and Algorithms. Chapman and Hall/CRC, 2012